# EUROPÄISCHE PATENTSCHRIFT

(11) **EP 0 766 660 B1**
(45) Veröffentlichungstag und Bekanntmachung des Hinweises auf die Patenterteilung: **29.12.1999**
(21) Anmeldenummer: 95923316.4
(22) Anmeldetag: 13.06.1995
(51) Int. Cl.: C07C 69/013, C07F 7/08, C07C 251/44, C07C 49/497, C07C 43/188, C07C 49/172, C07C 43/196

(54) **CYCLOHEXANON-DERIVATE, VERFAHREN ZU DEREN HERSTELLUNG UND ZWISCHENPRODUKTE DES VERFAHRENS**
CYCLOHEXANONE DERIVATIVES, METHOD OF PREPARING THEM AND INTERMEDIATES USED IN THE METHOD
DERIVES DE CYCLOHEXANONE, LEUR PROCEDE DE PREPARATION ET INTERMEDIAIRES DUDIT PROCEDE

(30) Priorität: 23.06.1994 DE 4423669
(43) Veröffentlichungstag der Anmeldung: 09.04.1997
(73) Patentinhaber: SCHERING AKTIENGESELLSCHAFT, 13353 Berlin (DE)
(72) Erfinder: STEINMEYER, Andreas, D-13581 Berlin (DE); NEEF, Günter, D-10711 Berlin (DE); MÜLLER, Gunnar, D-13409 Berlin (DE); KNÖLKER, Hans-Joachim, D-76344 Leopoldshafen (DE)
(86) Internationale Anmeldenummer: EP9502274
(87) Internationale Veröffentlichungsnummer: WO9600207

(56) Entgegenhaltungen:
- TETRAHEDRON LETTERS, Bd. 28, Nr. 46, 1987 OXFORD GB, Seiten 5723-5726, KEIJI MARUAKA ET AL. 'UNUSUAL CONJUGATE ADDITION OF ORGANOLITHIUM REAGENT TO ALPHA,BETA-UNSATURATED KETONES'

## Beschreibung

Die folgende Erfindung betrifft Cyclohexanon-Derivate der allgemeinen Formel **I**, 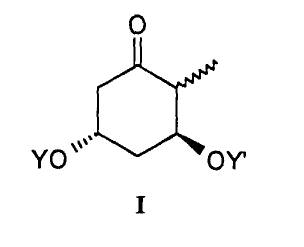 worin Y und Y' gleich oder verschieden sein können. Im Einzelnen können Y und Y' je ein Wasserstoffatom, eine Alkanoylgruppe mit 1 bis 9 Kohlenstoffatomen oder eine Aroylgruppe
oder je eine alkyl- oder aryl- oder gemischt aryl-alkylsubstituierte Silylgruppe oder eine andere gängige Hydroxylschutzgruppe (siehe T.W. Greene and P.G.M. Wuts, Protective Groups in Organic Synthesis (2nd Ed.), p. 10-118, Wiley, 1991) bedeuten. Vorzugsweise bedeuten Y und Y' die Acetyl-, Propionyl-, Pivaloyl- oder Benzoylgruppe bzw. die Trimethylsilyl- (TMS), Triethylsilyl- (TES), *tert*.-Butyldimetylsilyl- (TBDMS), *tert.*-Butyldiphenylsilyl- (TBDPS) oder Triisopropylsilylschutzgruppe (TIPS) bzw. die Methoxymethyl- (MOM), Methoxyethoxymethyl- (MEM), Ethoxyethyl- (EE), Trimethylsilylethoxymethyl- (SEM), Tetrahydrofuranyl- (THF) oder Tetrahydropyranylgruppe (THP).

Die vorliegende Erfindung betrifft weiterhin ein Verfahren zur Herstellung der Cyclohexanon-Derivate der allgemeinen Formel **I** und Zwischenprodukten zur Synthese von Vitamin D-A-Ring-Fragmenten und 1α-Hydroxy-Vitamin D-Derivaten.

Besonders bevorzugt sind folgende Cyclohexanon-Derivate:
(3*S*,5*S*)-3,5-Bis[[dimethyl(1,1-dimethylethyl)silyl]oxy]-2-methylcyclohexanon
(3*S*,5*S*)-3,5-Bis[[(1,1-dimethylethyl)diphenylsilyl]oxy]-2-methylcyclohexanon
(3*S*,5*S*)-3, 5-Bis[(triethylsilyl)oxy]-2-methylcyclohexanon
(3*S*,5*S*)-3,5-Bis(acetyloxy)-2-methylcyclohexanon
(3*S*,5*S*)-3,5-Bis(2,2-dimethyl-1-oxopropoxy)-2-methylcyclohexanon
(3*S*,5*S*)-3,5-Bis(benzoyloxy)-2-methylcyclohexanon
(3*S*,5*S*)-3,5-Bis(1-ethoxyethoxy)-2-methylcyclohexanon

Beansprucht werden weiterhin die folgenden Synthesezwischenprodukte sowie Verfahren zu deren Herstellung, 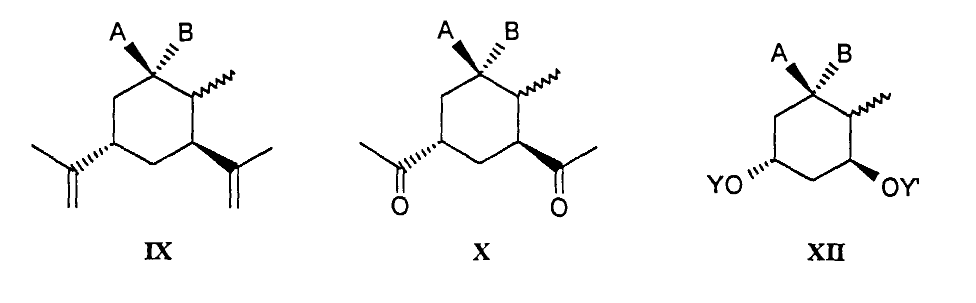 worin A und B gemeinsam eine freie oder geschützte Carbonylgruppe bedeuten (Schutzgruppen: Dialkoxyketal, 1,3-Dioxan, 1,3-Dioxolan, Dialkylhydrazon, Tosylhydrazon, Oxim, Alkyl- oder Benzyloximether) oder A oder B ein Wasserstoffatom und die entsprechende andere Gruppe B oder A eine Hydroxygruppe, welche frei oder geschützt sein kann (Schutzgruppen: Benzylether, p-Methoxybenzylether, o-, m-, p-Nitrobenzylether, TBDMS-Ether, TIPS-Ether, TBDPS-Ether, TES-Ether, MOM-Ether, MEM-Ether, SEM-Ether, EE-Ether, THP-Ether, THF-Ether o.ä.), bedeuten. Die Bedeutungen für Y und Y' entsprechen den für die allgemeine Formel **I** angegebenen Gruppen.

Besonders bevorzugt sind folgende Zwischenprodukte:
(1*S*,5*S*)-1,5-Bis(1-methylethenyl)-2-methyl-3-(phenylmethoxy)cyclohexan
(1*S*,3*S*)-1,'1-[4-Methyl-5-(phenylmethoxy)1,3-cyclohexandiyl]bis[ethanon]
(1*S*,5*R*)-1,5-Bis(acetyloxy)-2-methyl-3-(phenylmethoxy)cyclohexan
(1*S*,3*S*)-Methyl-5-(phenylmethoxy)1,3-cyclohexandiol
(1*S*,5*R*)-1,5-Bis[[dimethyl(1,1-dimethylethyl)silyl]oxy]2-methyl-3-(phenylmethoxy)cyclohexan
(3*S,*5*R*)-3,5-Bis[[dimethyl(1,1-dimethylethyl)silyl]oxy]-2-methylcyclohexanol
(3*S*,5*R*)-3,5-Bis(acetyloxy)-2-methylcyclohexanol
(*E*)-(3*S*,5*S*)-3,5-Bis(1-methylethenyl)-2-methylcyclohexanonoxim
(*Z*)-(3*S*,5*S*)-3,5-Bis(1-methylethenyl)-2-methylcyclohexanonoxim
(*E*)-(3*S*,5*S*)-3,5-Bis(1-methylethenyl)-2-methylcyclohexanon-O-methyloxim
(*Z*)-(3*S*,5*S*)-3,5-Bis(1-methylethenyl)-2-methylcyclohexanon-O-methyloxim
(*E*)-(1*S*,3*S*)-1,1'-[5-(Hydroxyimino)-4-methyl-1,3-cyclohexandiyl]bis[ethanon]
(*Z*)-(1*S*,3*S*)-1,1'-[5-(Hydroxyimino)-4-methyl-1,3-cyclohexandiyl]bis[ethanon]
(*E*)-(1*S*,3*S*)-1,1'-[5-(Methoxyimino)-4-methyl-1,3-cyclohexandiyl]bis[ethanon]
(*Z*)-(1*S*,3*S*)-1,1'-[5-(Methoxyimino)-4-methyl-1,3-cyclohexandiyl]bis[ethanon]
(*E*)-(3*S*,5*R*)-3,5-Bis(acetyloxy)-2-methylcyclohexanonoxim
(*E*)-(3*S*,5*R*)-3,5-Bis(acetyloxy)-2-methylcyclohexanon-O-methyloxim
(*Z*)-(3*S*,5*R*)-3,5-Bis(acetyloxy)-2-methylcyclohexanon-O-methyloxim
(*E*)-(3*S*,5*R*)-3,5-Dihydroxy-2-methylcyclohexanonoxim
(*Z*)-(3*S*,5*R*)-3,5-Dihydroxy-2-methylcyclohexanonoxim
(*E*)-(3*S*,5*R*)-3,5-Dihydroxy-2-methylcyclohexanon-O-methyloxim
(*Z*)-(3*S*,5*R*)-3,5-Dihydroxy-2-methylcyclohexanon-O-methyloxim
(*E*)-(3*S*,5*R*)-3,5-Bis[[dimethyl(1,1-dimethylethyl)silyl]oxy]-2-methylcyclohexanonoxim
(*Z*)-(3*S*,5*R*)-3,5-Bis[[dimethyl(1,1-dimethylethyl)silyl]oxy]-2-methylcyclohexanonoxim
(*E*)-(3*S*,5*R*)-3,5-Bis[[dimethyl(1,1-dimethylethyl)silyl]oxy]-2-methylcyclohexanon-O-methyloxim
(Z)-(3*S*,5*R*)-3,5-Bis[[dimethyl(1,1-dimethylethyl)silyl]oxy]-2-methylcyclohexanon-O-methyloxim
(1*S*,5*S*)-1,5-Bis(1-methylethenyl)-3-[[(1,1-dimethylethyl)diphenylsilyl]oxy]-2-methylcyclohexan
(1*S*,3*S*)-1,1'-[5-[[(1,1-Dimethylethyl)diphenylsilyl]oxy]-4-methyl-1,3-cyclohexandiyl]bis[ethanon]
(1*S*,5*S*)-1,5-Bis(acetyloxy)-3-[[(1,1-dimethylethyl)diphenylsilyl]oxy]-2-methylcyclohexan
(1*S*,3*S*)-5-[[(1,1-Dimethylethyl)diphenylsilyl]oxy]-4-methyl-1,3-cyclohexandiol
(1*S*,5*S*)-1,5-Bis(1-ethoxyethoxy)-3-[[(1,1-dimethylethyl)diphenylsilyl]oxy]-2-methylcyclohexan
(3*S*,5*R*)-3,5-Bis(-ethoxyethoxy)-2-methylcyclohexanol

Der Vorteil der vorliegenden Erfindung liegt darin, daß ausgehend von Carvon ein A-Ring-Fragment geschaffen wird, welches gleich im ersten Schritt in den 3- und 5-Positionen identische Substitutionsmuster enthält und alle anschließenden synthetischen Manipulationen an diesen Zentren simultan ablaufen können. Innerhalb des Standes der Technik sind bereits ähnliche Synthesen, ebenfalls ausgehend von Carvon, beschrieben, in denen die Zentren in den 3- und 5-Positionen mittels eines längeren, komplizierteren Syntheseweges aufgebaut werden, um zur trans-Diol-Struktur zu gelangen [Tetrahedron Letters, Vol. 28, 2099-2102 (1987) und J. Org. Chem. 54, 3515-3517 (1989)].

Alle Verbindungen der allgemeinen Formeln **I**, **IX**, **X** und **XII** lassen sich durch Standardreaktionen aus den entsprechenden Vorstufen herstellen; vergl. Reaktionsschema 1 und 2.
Derivate der allgemeinen Formel **I** können leicht in 1α-Hydroxy-Vitamin D-A-Ring-Fragmente der allgemeinen Formel **II** überführt werden, 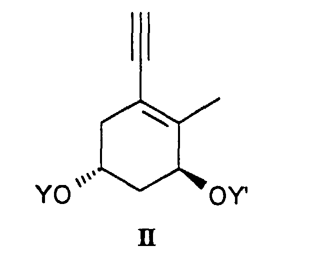 worin Y und Y' die bereits beschriebene Bedeutung haben. Verbindungen vom Typ **II** sind literaturbekannt und können mit geeigneten CD-Ring-Fragmenten und etablierter Folgechemie bekanntermaßen in 1α-Hydroxy-Vitamin D-Analoga überführt werden [B. Lythgoe et al. Tetrahedron Lett. 3649 (1973), J. Chem. Soc. Perk. I 2654 (1974), A. Mourino et al. Tetrahedron Lett. **29** 1203 (1988), W.H. Okamura et al. J. Org. Chem. **54** 4072 (1989) und EP 0 521 550 A2].

Primär erzeugt man dabei Eninen-Derivate vom Typ **III**, 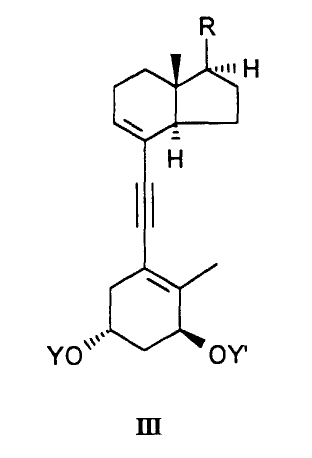 woraus durch Lindlar-Hydrierung der Dreifachbindung zur cis-Doppelbindung und anschließender thermisch induzierter 1,7-Wasserstoffverschiebung 1α-Hydroxy-Vitamin D-Derivate der allgemeinen Formel **IV** herstellbar sind. Der Rest R bedeutet dabei eine der natürlichen oder der literaturbekannten artifiziellen Vitamin D-Seitenketten.

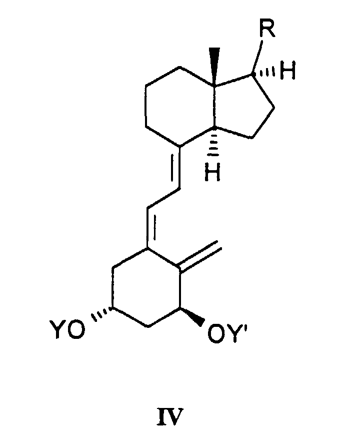

Somit stellt die vorliegende Erfindung ein neues Verfahren zur Synthese von Zwischenprodukten zur Darstellung von 1α-Hydroxy-Vitamin D-Analoga dar.
Diese können bei der Wahl geeigneter Seitenketten für die Herstellung von Arzneimitteln für die Behandlung bestimmter Krankheiten (Psoriasis, maligne Tumore, Akne, Autoimmunerkrankungen, Wundheilung, Osteoporose usw.) verwendet werden. Beispiele für entsprechende Derivate, deren biologische Wirkungen und die Zielindikationen sind in folgenden Patentanmeldungen dokumentiert:
Schering AG EP 421 561, WO 91/12238, DE 40 03 854, DE 41 01 953, DE 41 41 746, WO 93/12081, DE 42 20 757, WO 94/00428, DE 42 21 961 WO 94/00429, DE 42 34 382, DE 43 17 415, WO 94/07853
Die Überführung von **I** in **II** kann durch Umsetzung mit Lithium-, Natrium- oder Kaliumacetylid oder dem entsprechenden Mono-Trimethylsilylacetylid in eine Verbindung der allgemeinen Formel **V** 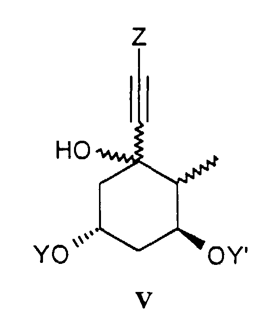 erreicht werden, wobei Z ein Wasserstoffatom oder die Trimethylsilylgruppe bedeutet. Durch anschließende Wassereliminierung (z.B. POCl₃, Pyridin; SOCl₂, Pyridin; Erhitzen mit wasserfreiem Kupfersulfat; Burgess-Reagenz; Einwirken von Diethylaminoschwefeltrifluorid; Einwirken von Säure wie Mineralsäuren, Essigsäure, Oxalsäure, Trifluoressigsäure) oder Überführung der Hydroxylgruppe in eine Fluchtgruppe mit anschließender Eliminierung (z.B. Mesylat oder Tosylat und Baseneinwirkung; Xanthogenat oder Acetat und Pyrolyse) gefolgt von möglicher Trennung der Regioisomeren erhält man **II**.
Eine andere Strategie beinhaltet die Umsetzung von **I** zu einem thermodynamisch kontrollierten Enolderivat der allgemeinen Formel **VI** (z.B. Enoltriflat X=OSO₂CF₃; Enolnonaflat X=OSO₂C₄F₉; Vinylhalogenid X=F, Cl, Br, I) und Kupplung mit einem geeigneten Acetylen (z.B. Trimethylsilylacetylen) bzw. die Umsetzung von den entsprechenden Vinylstannanen (X=Alkyl₃Sn) oder Vinylboronsäuren oder deren Ester (X=B(OH)₂, B(OR)₂) mit Halogenacetylen in Palladium-katalysierten Reaktionen zu **II** [K. Ritter Synthesis 735 (1993), S. Cacchi Synthesis 320 (1986), L. Brandsma Synth. Comm. **20**, 1889 (1990), N. Miyaura et al. Tetrahedron Lett. **22**, 127 (1981)]. Hier eröffnet sich auch die Möglichkeit, das erwähnte Enolderivat **VI**, wobei X=OSO₂CF₃; X=OSO₂C₄F₉; X=F, Cl, Br, I bedeuten können, direkt mit einem geeignet substituierten Vitamin D-CD-Fragment [z.B. **VII**, W.H. Okamura et al. J. Org. Chem. **49** 2152 (1984)] zu kuppeln.

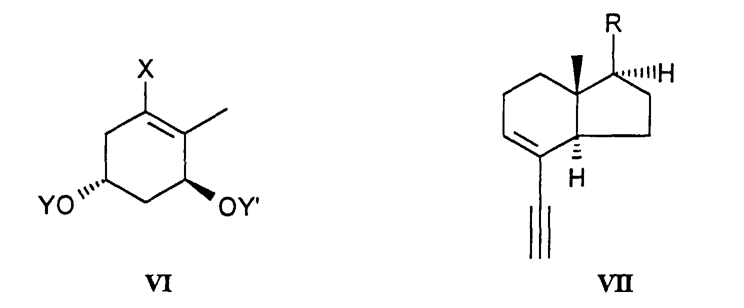

Die Synthese der erfindungsgemäßen Verbindungen der allgemeinen Formel **I** erfolgt auf einem neuen, sehr direkten Weg ausgehend von (*S*)-Carvon (kommerziell erhältlich), welches in einer konjugierten Addition mit einer geeigneten metallorganischen Verbindung (Isopropenyl-Cuprat, Isopropenylmagnesiumhalogenide unter Kupferkatalyse) in die Verbindung **VIII** überführt wird.

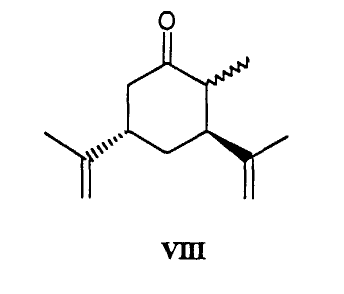

Die Einführung der Isopropenylgruppe erfolgt ganz selektiv von der β-Seite des Moleküls, wobei die für das Calcitriol-Derivat geforderte absolute und relative Konfiguration in der späteren 1- und 3-Position aufgebaut wird. Ein Vorteil dieses Approaches ist darüberhinaus die gleichartige Substitution dieser Zentren, so daß alle chemische Manipulation simultan durchgeführt werden können.
Die Ketogruppe in **VIII** wird nun unter Standardbedingungen als Ketal, Hydrazon o.ä. geschützt oder mit einem Reduktionsmittel (z.B. NaBH₄, LiAlH₄, DIBAH, Superhydrid, Selectride, Meerwein-Ponndorf-Bedingungen, Borane) zum Alkohol reduziert, welcher anschließend mit einer Schutzgruppe versehen wird, die die nachfolgenden Reaktionsbedingungen tolerieren muß (z.B. Benzylether, auch arylsubstituiert; TBDPS-Ether; TBDMS-Ether usw.), wobei Derivate der allgemeinen Formel **IX** entstehen, 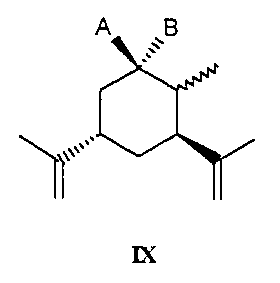 worin A und B die bereits zuvor beschriebenen Bedeutungen haben.

Nachfolgend werden die beiden Vinylgruppen zu den korrespondierenden Methylketonen der allgemeinen Formel **X** abgebaut.

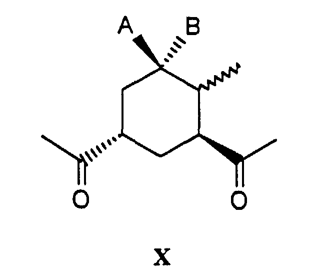

Für diese Reaktion kommt eine Ozonolyse mit mild reduktiver Aufarbeitung (Triphenylphosphin, Dimethylsulfid, Amine) infrage. Möglich ist auch die Dihydroxylierung der Doppelbindungen unter Standardbedingungen (z.B. KMnO₄; OsO₄, Reoxidationsmittel) gefolgt von Glykolspaltung (z.B. NaIO₄; HIO₄; Pb(OAc)₄). In einer doppelten Baeyer-Villiger Reaktion wird X dann in das Diacetat **XI** überführt.

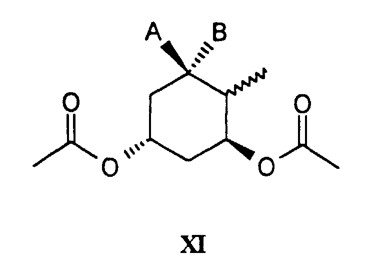

Eine Übersicht über die Vielzahl der anwendbaren Methoden gibt ein Review-Artikel: G.R. Krow Org. React. **43**, 251 (1993). Hervorzuheben ist eine neuartige Methode, die ohne die Verwendung von hochkonzentrierten Oxidationsmitteln wie 90% Wasserstoffperoxid oder 100% MCPBA auskommt: Harnstoff/H₂O₂-Addukt, Trifluoressigsäureanydrid [M.S. Cooper, H. Heaney, A.J. Newbold, W.R. Sanderson Synlett 533 (1990)]. Diese Methode erscheint speziell für die industrielle Anwendung besonders geeignet und liefert tatsächlich im hier gezeigten Fall die besten Ergebnisse. Anschließend können die Acetate zu den freien Hydroxy-Gruppen gespalten und andere Schutzgruppen eingeführt werden (z.B. TBDMS, EE), wobei die Verbindung der allgemeinen Formel **XII** erhalten wird. Die Acetate können aber auch beibehalten werden.

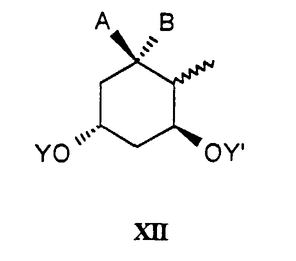

Die Definitionen für A, B und Y sowie Y' wurden schon früher gegeben.
Falls A und B eine Carbonylschutzgruppe bedeuten, so führt die Spaltung dieser Gruppe (z.B. Ketal: Säurekatalyse; Oximalkylether: TiCl₃, DIBAH oder Natriumbisulfit; Oximbenzylether: Hydrogenolyse) direkt zur Verbindung der allgemeinen Formel **I**, während für A=Wasserstoff und B=Hydroxylschutzgruppe oder umgekehrt die entsprechende Spaltung (Silylgruppen: TBAF, HF oder HF/Pyridin; Benzylether: Hydrogenolyse) die Verbindung der allgemeinen Formel **XIII** liefert.

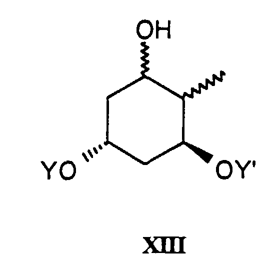

Die Umsetzung zu **I** erfolgt dann durch Oxidation mit einem geeigneten Oxidationsmittel z.B. RuO₂NaIO₄ [K. Sakai Tetrahedron Asymmetry **3**, 297 (1992)] oder Swern-Bedingungen. Alternativ sind auch andere Oxidationsmethoden denkbar: z.B. PCC, PDC, Dess-Martin-Reagenz, BaMnO₄.

Durch das beschriebene neue Verfahren wird ausgehend von kommerziell erhältlichem (*S*)-Carvon ein 1α-Hydroxy-Vitamin D-A-Ring-Fragment vom Typ **II** dargestellt, wobei alle chemischen Manipulationen sehr leicht durchführbar und problemlos in den Produktionsmaßstab übertragbar sind.
Die Gesamtausbeute der Sequenz liegt ohne aufwendige Optimierungsversuche zwischen 16 und 18% über 11 Stufen. Somit bietet das vorliegende Verfahren erhebliche Vorteile gegenüber den in der Literatur beschriebenen Synthesen von **II** oder entsprechenden Precursor-Derivaten [A. Mourino et al. Tetrahedron Lett. **28**, 2099 (1987), M.R. Uskokovic et al. Tetrahedron Lett. **28,** 2095 (1987), S. Takano et al. J. Org. Chem. **54,** 3515 (1989)]. Diese Verfahren sind aufgrund der zum Teil schlechten Ausbeuten sowie der Verwendung aufwendiger Reaktionsbedingungen oder kostspieliger, umweltbelastender Reagenzien allenfalls von akademischem Interesse.
Die nachfolgenden Beispiele dienen der Erläuterung der Erfindung.

### Beispiele:

### (3S,5S)-3,5-Bis(1-methyletheny)-2-methylcyclohexanon 1

Zu einer Lösung aus 9,68 g (80 mmol) 2-Brom-1-propen in 160 ml absolutem Diethylether werden bei -78°C 95 ml einer 1,7 M *tert*.-Butyllithiumlösung in Pentan (160 mmol) langsam zugetropft, und es wird 1 Stunde bei der gegebenen Temperatur nachgerührt. Anschließend werden 3,58 g (40 mmol) Kupfer(I)-Cyanid zugesetzt, und das Reaktionsgemisch wird langsam auf -20°C erwärmt sowie 30 Minuten bei dieser Temperatur gehalten. Letztendlich wird das Reaktionsgemisch erneut auf -78°C abgekühlt und mit 4,51 g (30 mmol) (*S*)-Carvon versetzt.
Anschließend wird das Reaktionsgemisch langsam auf Raumtemperatur erwärmt und vorsichtig mit gesättigter Ammoniumchloridlösung gequencht. Nach dem Verdünnen mit Essigsäureethylester wird noch dreimal mit gesättigter Ammoniumchloridlösung gewaschen und die vereinigten wäßrigen Phasen noch zweimal mit Essigsäureethylester reextrahiert. Die vereinigten organischen Phasen werden mit gesättigter Natriumchloridlösung gewaschen, über Natriumsulfat getrocknet, filtriert und das Lösungsmittel am Rotationsverdampfer abgezogen. Der Rückstand wird über eine Kieselgelsäule aufgereinigt (Laufmittel: EE:H = 1:9; R_{f}=0,8).
Man erhält 5,48 g (28,47 mmol) der Titelverbindung **1** als farblose Flüssigkeit.
Generell werden die spektroskopischen Daten des Hauptdiastereomeren angegeben.
¹H-NMR (300 MHz, CDCl₃): δ= s 4,95 ppm (1H); s 4,80 ppm (1H); s 4,75 ppm (1H); s 4,525 ppm (1H); m 2,75-2,55 ppm (3H); dd 2,525 ppm [5+1]Hz (1H); ddd 2,30 ppm [15+10+1]Hz (1H); m 2,00 ppm (1H); dd 1,825 [10+5] (1H); s 1,725 ppm (3H); s 1,70 ppm (3H); d 1,075 ppm [7,5] (3H)
MS (EI): m/z= 192 (M⁺)[3%]; 177 (M⁺)[2%]; 95 [32%]; 83 [100%]
IR (CHCl₃): ν = 1701, 1451, 1377, 901 cm⁻¹

### Darstellung von 1 durch Grignard-Reaktion

Man legt 5,4 g (220 mmol) Magnesiumspäne in 60 ml THF vor und tropft 25,41 g (210 mmol) 2-Brompropen in 200 ml THF zu, so daß die Mischung zum leichten Sieden kommt. Es wird 15 Min. nachgerührt und dann auf 0°C gekühlt. Man fügt nun 396 mg (4 mmol) Kupfer(I)-Chlorid hinzu, rührt weitere 15 Min. bei 0°C und addiert schließlich 22,53 g (150 mmol) (*S*)-Carvon in 100 ml THF. Die Reaktionsmischung wird nun langsam auf Raumtemperatur erwärmt und analog der obigen Cuprat-Addition aufgearbeitet, wobei man 25,38 g (132 mmol) der Titelverbindung **1** als farblose Flüssigkeit erhält.

### (3S,5S)-3,5-Bis(1-methylethenyl)-2-methylcyclohexanol 2

Zu einer Lösung aus 5,47 g (28,40 mmol) 1 in 150 ml absolutem Methanol werden bei Raumtemperatur portionsweise 1,14 g (30 mmol) Natriumborhydrid zugesetzt, und das Reaktionsgemisch wurde 4 Stunden nachgerührt.
Zur Aufarbeitung wird mit Wasser hydrolysiert, mit Dichlormethan mehrfach extrahiert sowie die vereinigten organischen Phasen mit gesättigter Natriumchloridlösung gewaschen, über Natriumsulfat getrocknet und das Lösungsmittel am Rotationsverdampfer abgezogen. Der Rückstand wird über eine Kieselgelsäule aufgereinigt (Laufmittel: EE:H = 2:8; R_{f}=0,2).
Man erhält 4,55 g (23,43 mmol) der Titelverbindung **2** als farblose Flüssigkeit.
¹H-NMR (300 MHz, CDCl₃): δ= q 4,90 ppm [1]Hz (1H); m 4,85 ppm (2H); s 4,65 ppm (1H); ddd 4,00 [12,5+5+5]Hz (1H); s(br) 2,50 ppm (1H); m 2,2 ppm (2H); dm 1,95 ppm [12,5]Hz (1H); s 1,725 ppm (3H); s 1,70 ppm (3H); m 1,65 ppm (4H); d 0,75 ppm [7,5]Hz (3H)
MS (EI): m/z= 194 (M⁺)[5%]; 177 (M⁺-OH)[47%]; 176 (M⁺-H₂O)[48%]; 133 [80%]; 107 [81%]; 93 [45%]

### Darstellung von 2 durch DIBAH-Reduktion von 1

Man legt 19,2 g (100 mmol) **1** in 400 ml Toluol vor und tropft bei -78°C 100 ml (120 mmol) DIBAH in Toluol zu. Man erwärmt langsam auf Raumtemperatur, gibt Isopropanol/Wasser hinzu und rührt 30 Min. nach. Der Niederschlag wird abfiltriert und das Filtrat mit Natriumchloridlösung gewaschen, über Natriumsulfat getrocknet und eingeengt. Der Rückstand wird an Kieselgel chromatographiert (Laufmittel: EE:H = 2:8), wobei 17,49 g (90,03 mmol) der Titelverbindung **2** als farblose Flüssigkeit anfallen.

### (1S,5S)-1,5-Bis(1-methylethenyl)-2-methyl-3-(phenylmethoxy)cyclohexan 3

Zu einer Lösung aus 4,50 g (23,16 mmol) **2** in 60 ml THF werden bei Raumtemperatur 1,8 g (60 mmol) einer 80%-igen Natriumhydridsuspension portionsweise zugesetzt, und es wird 1 Stunde nachgerührt. Anschließend werden 10,26 g (60 mmol) Benzylbromid bei der gegebenen Temperatur zugetropft. Zusätzlich wird eine Spatelspitze Dimethylaminopyridin zugesetzt und das Reaktionsgemisch über Nacht bei Raumtemperatur gerührt.
Zur Aufarbeitung wird mit Wasser hydrolysiert und mit Hexan extrahiert. Die vereinigten organischen Phasen werden mit gesättigter Natriumchloridlösung gewaschen, über Natriumsulfat getrocknet, filtriert und das Lösungsmittel am Rotationsverdampfer abgezogen. Der Rückstand wird über eine Kieselgelsäule aufgereinigt (Laufmittel: Hexan; R_{f}=0,9).
Man erhält 5,08 g (17,87 mmol) der Titelverbindung **3** als farblose Flüssigkeit.
¹H-NMR (300 MHz, CDCl₃): δ= m 7,35 ppm (5H); dd 4,875 [7,5+2,5]Hz (1H); s 4,75 ppm (1H); s 4,65 ppm (1H); dd 4,6 ppm [7,5+2,5]Hz (1H);s 4,55 ppm (2H); ddd 3,7 ppm [7,5+5+5]Hz (1H); s(br) 2,55 ppm (1H); m 2,45 ppm (1H); m 2,15 ppm (1H); m 2,05 ppm (1H); m 1,75 ppm (9H); d 0,80 ppm [7,5]Hz (3H)
MS (EI): m/z= 284 (M⁺) [7%]; 282 [25%]; 193 (M⁺-Bn)[7%]; 91 (Bn)[100%]

### (1S,3S)-1,1'-[4-Methyl-5-(phenylmethoxy)-1,3-cyclohexandiyl]bis[ethanon] 4

Zu einer Lösung aus 5,37 g (18,87 mmol) **3** in 100 ml absolutem Dichlormethan wird bei -78°C ca. 30 Min. lang ein schwacher Ozongasstrom durchgeleitet. Nach Beendigung der Reaktion (DC-Kontrolle) werden 10,5 g (40 mmol) Triphenylphosphin hinzugefügt und das Reaktionsgemisch wird langsam auf Raumtemperatur erwärmt.
Zur Aufarbeitung wird mit Dichlormethan verdünnt, mit Wasser gewaschen, die vereinigten wäßrigen Phasen mit Dichlormethan reextrahiert sowie die vereinigten organischen Phasen mit gesättigter Natriumchloridlösung gewaschen, über Natriumsulfat getrocknet, abfiltriert und das Lösungsmittel am Rotationsverdampfer abgezogen. Der Rückstand wird über eine Kieselgelsäule aufgereinigt (Laufmittel: EE:H = 8:2; R_{f}=0,1). Man erhält 2,29 g (7,94 mmol) der Titelverbindung **4** als farblose Flüssigkeit.
¹H-NMR (300 MHz, CDCl₃): δ= m 7,3 ppm (5H); d 4,6 ppm [12]Hz (1H); d 4,5 ppm [12]Hz (1H); ddd 3,65 ppm [7,5+5+5]Hz (1H); m 2,85 ppm (1H); m 2,65 ppm (2H); s 2,12 ppm (3H); s 2,10 ppm (3H); m 1,9-1,6 ppm (4H); d 0,85 ppm [7,5]Hz (3H) MS (EI): m/z= 288 (M⁺)[1%]; 245 (M⁺-CH₃CHO)[5%]; 197 (M⁺-Bn)[4%]; 91 (Bn)[100%)]
IR(CHCl₃): ν=3034, 1706, 1357,1262, 1095 cm⁻¹

### (1S,5R)-1,5-Bis(acetyloxy)-2-methyl-3-(phenylmethoxy)cyclohexan 5

Zu einer Lösung aus 2,28 g (7,9 mmol) **4** in 160 ml absolutem Dichlormethan werden bei Raumtemperatur 20,95 g (154 mmol) Kaliumdihydrogenphosphat sowie 15,05 g (160 mmol) Harnstoff/H₂O₂-Addukt (Merck) gegeben. Anschließend werden 8,40 g (40 mmol) Trifluoressigsäureanhydrid langsam zugetropft. Es wird 2 Stunden bei 40°C nachgerührt.
Zur Aufarbeitung wird mit gesättigter Natriumhydrogencarbonat gequencht, mit Dichlormethan verdünnt, die organische Phase je einmal mit gesättigter Natriumsulfitlösung, Wasser und gesättigter Natriumchloridlösung gewaschen, über Natriumsulfat getrocknet, abfiltriert und das Lösungsmittel am Rotationsverdampfer abgezogen. Man erhält zwei Produkte, die über eine Kieselgelsäule getrennt werden (Laufmittel: EE:H = 4:6; F₁= 1,32 g (4,11 mmol) R_{f1}=0,6 (Diacetat); F₂= 0,53 g (1,74 mmol) R_{f2}=0,5 (Monoacetat). Das Monoacetat wurde der obigen Prozedur erneut unterworfen.
Spektroskopische Daten für **5**:
¹H-NMR (300 MHz, CDCl₃): δ= m 7,35 ppm (5H); m 5,2 ppm (1H); m 5,1 ppm (1H); d 4,55 ppm [12]Hz (1H); d 4,5 ppm [12]Hz (1H); ddd 3,8 ppm [7,5+5+5]Hz (1H); m 2,6 ppm (1H); s 2,1 ppm (3H); s 2,05 ppm (3H); m 1,85 ppm (4H); d 0,95 ppm [7,5]Hz (3H)
MS (EI): m/z= 200 [3%]; 169 [5%]; 109 [25%]; 91 (Bn)[100%]
IR (CHCl₃): ν= 1730, 1372, 1252, 1028 cm⁻¹

### (1S,3S)-4-Methyl-5-(phenylmethoxy)-1,3-cyclohexandiol 6

Zu einer Lösung aus 1,15 g (3,16 mmol) **5** in 10 ml absolutem Methanol werden bei Raumtemperatur 378 mg (7 mmol) Natriummethanolat zugegeben und es wird 24 Stunden bei der gegebenen Temperatur gerührt.
Zur Aufarbeitung wird mit gesättigter Ammoniumchloridlösung neutralisiert und mit Dichlormethan extrahiert. Die vereinigten organischen Phasen werden mit gesättigter Natriumchloridlösung gewaschen, über Natriumsulfat getrocknet, abfiltriert und das Lösungsmittel am Rotationsverdampfer abgezogen. Der Rückstand wird über eine Kieselgelsäule aufgereinigt (Laufmittel: EE ; R_{f}=0,3).
Man erhält 558 mg (2,36 mmol) der Titelverbindung **6** als kristalline Substanz.
¹H-NMR (300 MHz, CDCl₃): δ= m 7,35 ppm (5H); d 4,7 ppm [12]Hz (1H); d 4,4 ppm [12]Hz (1H); m 4,25 ppm (1H); m 3,9 ppm (1H); s(br) 3,8 ppm (1H); m 2,5 ppm (1H); m 2,3 ppm (2H); m 1,8-1,3 ppm (4H); d 1,2 ppm [7,5]Hz (3H)
MS (EI): m/z= 218 (M⁺-H₂O)[2%]; 107 [12%]; 91 (Bn)[100%]

### (1S,5R)-1,5-Bis[[dimethyl(1,1-dimethylethyl)silyl]oxy]-2-methyl-3-(phenylmethoxy)-cyclohexan 7

Zu einer Lösung aus 1,51 g (10 mmol) TBDMSC1 und 1,36 g (20 mmol) Imidazol in 10 ml absolutem DMF werden bei Raumtemperatur 557 mg (2,36 mmol) **6** in 5 ml absolutem DMF zugetropft, und es wird über Nacht bei der gegebenen Temperatur gerührt.
Zur Aufarbeitung wird mit Hexan verdünnt, mit Wasser hydrolysiert und mit gesättigter Ammoniumchloridlösung ausgeschüttelt. Die vereinigten wäßrigen Phasen werden mehrfach mit Hexan reextrahiert. Die vereinigten organischen Phasen werden mit gesättigter Natriumchloridlösung gewaschen, über Natriumsulfat getrocknet, filtriert und das Lösungsmittel wird am Rotationsverdampfer abgezogen. Der Rückstand wird über einer Kieselgelsäule aufgereinigt (Laufmittel: Hexan; R_{f}= 0,9).
Es werden 832 mg (1,79 mmol) der Titelverbindung 7 als farblose Flüssigkeit erhalten. ¹H-NMR (300 MHz, CDCl₃): δ= m 7,4 ppm (5H); d 4,6 ppm [12]Hz (1H); d 4,45 ppm [12]Hz (1H); m 4,1 ppm (2H); ddd 3,8 ppm [10+5+5]Hz (1H); m 2,4 ppm (1H); m 1,7 ppm (1H); m 1,55 ppm (1H); d 0,95 ppm [7,5]Hz (3H); s 0,9 ppm (9H); s 0,85 ppm (9H); 4x s 0,75-0,5 ppm (12H)
MS (EI): m/z= 449 (M⁺-Me)[1%]; 407 [57%]; 318 [38%]; 199 [28%]; 157 [19%]; 91 [100%]

### (3S,5R)-3,5-Bis[[dimethyl(1,1-dimethylethyl)silyl]oxy]-2-methylcyclohexanol 8

Zu einer Lösung aus 1,14 g (2,45 mmol) 7 in 60 ml absolutem Ethanol wird eine Spatelspitze 10%-iges Pd/C gegeben. Der Reaktionskolben wird mit einem Wasserstoffballon versehen, und es wird über Nacht gerührt.
Zur Aufarbeitung wird abfiltriert und der Filterkuchen gründlich mit Ethanol gespült. Abschließend wird das Lösungsmittel am Rotationsverdampfer eingeengt und der Rückstand über eine Kieselgelsäule aufgereinigt (Laufmittel: EE:H = 3 :7; R_{f}= 0,6). Man erhält 735 mg (1,96 mmol) der Titelverbindung **8** als farblose Flüssigkeit.
¹H-NMR (300 MHz, CDCl₃): δ= tt 4,3 ppm [12+4]Hz (1H); m 4,02 ppm (1H); d(br) 3,82 ppm [9]Hz (1H); m 2,32 (1H); m 2,05 ppm (1H); m 1,48 ppm (1H); d 1,10 ppm [7,5]Hz (3H); s 0,9 ppm (9H); s 0,88 ppm (9H); 4x s 0,75-0,5 ppm (12H)
MS (EI): m/z= 317 (M⁺-Bu)[7%]; 241 (M⁺-tBDMSiOH)[8%]; 227 [30%]; 199 [75%]; 185 [81%]; 159 [50%]; 157 [47%]; 115 [30%]; 75 [99%]; 73 [100%]

### (3S,5S)-3,5-Bis[[dimethyl(1,1-dimethylethyl)silyl]oxy]-2-methylcyclohexanon 9

Zu einer Lösung aus 444 mg (3,5 mmol) Oxalylchlorid in 20 ml absolutem Dichlormethan werden bei -78°C 547 mg (7 mmol) DMSO langsam zugetropft. Man läßt 20 Minuten bei der gegebenen Temperatur nachrühren. Anschließend werden 592 mg (1,58 mmol) **8** in 5 ml absolutem Dichlormethan hinzugefügt, und es wird erneut 15 Minuten nachgerührt. Danach wurden 2,83 g Triethylamin hinzugegeben, und das Reaktionsgemisch wird langsam auf Raumtemperatur erwärmt.
Zur Aufarbeitung wird Dichlormethan zugegeben und mit verdünnter HCI angesäuert. Die wäßrige Phase wird noch zweimal mit Dichlormethan reextrahiert, die vereinigten organischen Phasen mit gesättigter Natriumchloridlösung gewaschen, über Natriumsulfat getrocknet, abfiltriert und das Lösungsmittel am Rotationsverdampfer abgezogen. Der Rückstand wird über eine Kieselgelsäule chromatographiert (Laufmittel: EE:H = 2:8; R_{f}=0,4).
Man erhält 550 mg (1,48 mmol) der Titelverbindung **9** als weiße, kristallinen Substanz.
¹H-NMR (300 MHz, CDCl₃): δ= m 4,3 ppm (1H); m 4,15 ppm (1H); ddd 2,7 ppm [15+5+2]Hz (1H); m 2,4 ppm (2H); m 2,2 ppm (1H); m 1,8 ppm (1H); d 1,05 ppm [7,5]Hz (3H); s 0,9 ppm (9H); s 0,85 ppm (9H); s 0,5 ppm (12H)
MS (EI): m/z= 315 (M⁺-Bu)[27%]; 199 [5%]; 157 [100%]; 133 [8%]; 115 [13%]; 75 [16%]; 73 [22%]
IR (CHCl₃): ν= 2957, 2931, 2886, 2858, 1713, 1472, 1257,1097, 1004, 838 cm⁻¹

### (3S,5S)-3,5-Bis[[dimethyl(1,1-dimethylethyl)silyl]oxy]-1-ethinyl-2-methylcyclohexanol 10

Bei 0°C wird Acetylen für 20 Minuten in 50 ml THF eingeleitet. Man tropft 1,25 ml *n*-Butyllithium-Lösung (1,6 M in Hexan, 2 mmol) hinzu und rührt 20 Minuten bei dieser Temperatur nach. Anschließend werden 215 mg (0,58 mmol) **9** in 2 ml THF hinzugetropft und 2 Stunden gerührt. Danach wird mit gesättigter Ammoniumchloridlösung gequencht, mit Essigester verdünnt und die organische Phase mit gesättigter Natriumchloridlösung gewaschen, über Natriumsulfat getrocknet und das Solvens entfernt. Der Rückstand wird säulenchromatographisch an Kieselgel aufgereinigt (Laufmittel: EE:H=4:6, R_{f}=0,6), wobei man 163 mg (0,41 mmol) der Titelverbindung **10** als weiße kristallinen Substanz erhält.
¹H-NMR (300 MHz, CDCl₃): δ= m 4,22 ppm (1H); m 4,06 ppm (1H); ddd 2,48 ppm [15+4+2]Hz (1H); dbr 2,09 ppm (1H); m 1,69 ppm (2H); m 1,58 ppm (1H); d 1,25 ppm [7,5]Hz (3H); s 0,92 ppm (9H); s 0,89 ppm (9H); s 0,1 ppm (12H)

### (3S,5R)-3,5-Bis[[dimethyl(1,1-dimethylethyl)silyl]oxy]-1-ethinyl-2-methyl-1-cyclohexen 11

Man legt 90 mg (0,23 mmol) **10** in 8 ml Pyridin vor und fügt unter Argon 50 µl Thionylchlorid zu. Es wird für 10 Min. bei Raumtemperatur gerührt, auf Eiswasser gegossen und mit Essigester extrahiert. Die organische Phase wäscht man mit Natriumhydrogencarbonatlösung und Natriumchloridlösung, trocknet über Natriumsulfat, entfernt das Solvens und reinigt das Rohprodukt durch Chromatograhie an Kieselgel (Laufmittel: EE:H= 1:19; R_{f}=0,9), wobei man 71 mg (0,20 mmol) der Titelverbindung **11** als farbloses Öl erhält.
¹H-NMR (300 MHz, CDCl₃): δ= m 4,21 ppm (1H); m 4,09 ppm (1H); s 3,06 (1H); sbr 1,93 ppm (3H); s 0,91 ppm (9H); s 0,89 ppm (9H); s 0,6 ppm (12H)
IR (KBr): ν= 3310, 2085, 1260, 835 cm⁻¹
MS (EI): m/z= 380 (M⁺)[3%]; 365 (M⁺-Me)[25%]; 323 [80%]; 248 [100%]

### (3S,5R)-3,5-Bis(acetyloxy)-2-methylcyclohexanol 12

Zu einer Lösung aus 60 mg (0,19 mmol) **5** in 10 ml absolutem Ethanol wird eine Spatelspitze 10%-iges Pd/C gegeben. Der Reaktionskolben wird mit einem Wasserstoffballon versehen, und es wird über Nacht gerührt.
Zur Aufarbeitung wird abfiltriert und der Filterkuchen gründlich mit Ethanol gespült. Abschließend wird das Lösungsmittel am Rotationsverdampfer eingeengt und der Rückstand über eine Kieselgelsäule aufgereinigt (Laufmittel: EE:H = 1:1; R_{f}= 0,3).
Man erhält 44 mg (0,19 mmol) der Titelverbindung **12** als farbloses Öl.
¹H-NMR (300 MHz, CDCl₃): δ= m 5,2 ppm (2H); s(br) 4,0 ppm (1H); s 2,1 ppm (3H); m 2,05 ppm (3H); s 2,025 ppm (3H); m 1,65 ppm (3H); d 1,00 ppm [7,5]Hz (3H)

### (3S,5S)-3,5-Bis(acetyloxy)-2-methylcyclohexanon 13

Zu einer Lösung aus 50 mg (0,22 mmol) **12** in 1 ml Tetrachlormethan werden bei Raumtemperatur 25 mg Rutheniumdioxid gegeben. Die Suspension wird mit 0,85 ml einer 0,28 molaren Natriummetaperiodatlösung (0,24 mmol) in Wasser versetzt. Nach 2 Stunden Rühren bei der gegebenen Temperatur werden weitere 0,5 ml der wäßrigen Natriummetaperiodatlösung (0,14 mmol) hinzugefügt, und es wird erneut 2 Stunden gerührt.
Zur Aufarbeitung wird der Katalysator abfiltriert, die Suspension mit Essigsäureethylester verdünnt und der Filterkuchen gründlich mit Essigsäureethylester nachgewaschen. Die vereinigten organischen Phasen werden mit gesättigter Natriumchloridlösung gewaschen, über Natriumsulfat getrocknet, filtriert und das Lösungsmittel am Rotationsverdampfer abgezogen.
Man erhält 73,4 mg der Titelverbindung **13** als farbloses Öl.
¹H-NMR (300 MHz, CDCl₃): δ= m 5,4 ppm (1H); m 5,2 ppm (1H); ddd 2,9 ppm [15+5+2,5]Hz (1H); m 2,6 ppm (1H); m 2,45 ppm (2H); m 2,05 ppm (1H); s 2,05 ppm (6H); d 1,05 ppm [7,5]Hz (3H)

### (1S,5S)-1,5-Bis(1-methylethenyl)-3-[[(1,1-dimethylethyl)diphenylsilyl]oxy]-2-methylcyclohexan 14

Man legt 7,67 g (39,6 mmol) **2** in 50 ml DMF vor und addiert 6,54 g (96 mmol) Imidazol und 12,5 ml (48 mmol) *tert*.-Butyldiphenylsilylchlorid in 80 ml DMF. Es wird über Nacht bei Raumtemperatur gerührt und anschließend mit Natriumchloridlösung gequencht, mit Essigester extrahiert, die organische Phase mit Natriumchloridlösung gewaschen und über Natriumsulfat getrocknet. Nach dem Einengen chromatographiert man den Rückstand an Kieselgel (Laufmittel: EE:H = 2:8), wobei 17,05 g (39,4 mmol) der Titelverbindung **14** als farblose Flüssigkeit anfallen.
¹H-NMR (300 MHz, CDCl₃): δ= m 7,70 ppm (4H); m 7,39 ppm (6H); s 4,78 ppm (1H); s 4,60 ppm (1H); s 4,52 ppm (1H); s 4,13 ppm (1H); m 3,95 ppm (1H); m 2,33 ppm (1H); m 2,08 ppm (1H); m 1,92 ppm (1H); m 1,75 ppm (2H); m 1,59 ppm (2H); s 1,59 ppm (3H); s 1,42 ppm (3H); s 1,09 ppm (9H); d 0,81 ppm [7]Hz (3H)

### (1S,3S)-1,1'-[5-[[(1,1-Dimethylethyl)diphenylsilyl]oxy]-4-methyl-1,3-cyclohexandiyl]bis[ethanon] 15

Man löst 25,75 g (59,5 mmol) **14** in 420 ml Dichlormethan und 140 ml Methanol und leitet bei -78°C Ozon/Sauerstoffgemisch (Ozongenerator) ein bis die Lösung leicht blau gefärbt ist. Überschüssiges Ozon wird mit Stickstoff vertrieben und anschließend mit 9,32 g (150 mmol) Dimethylsulfid versetzt. Man läßt aufRaumtemperatur erwärmen, engt die Mischung ein und chromatographiert den Rückstand an Kieselgel (Laufmittel EE:H = 6:4), wobei man 41,1 g (55,19 mmol) der Titelverbindung **15** als farblose Flüssigkeit erhält.
¹H-NMR (300 MHz, CDCl₃): δ= m 7,69 ppm (4H); m 7,40 ppm (6H); m 3,82 ppm (1H); m 2,64 ppm (1H); dt 2,58 ppm [12+4]Hz (1H); m 2,27 ppm (1H); s 1,98 ppm (3H); m 1,78 ppm (3H); s 1,70 ppm (3H); 1,61 ppm (1H); s 1,09 ppm (9H); d 0,83 ppm [7]Hz (3H)

### (1S,5S)-1,5-Bis(acetyloxy)-3-[[(1,1-dimethylethyl)diphenylsilyl]oxy]-2-methylcyclohexan 16

Analog Beispiel **5** setzt man 17,68 g (40,5 mmol) **15** um, wobei 16,99 g ( 36,25 mmol) der Titelverbindung **16** als farbloses Öl anfallen.
¹H-NMR (300 MHz, CDCl₃): δ= m 7,68 ppm (4H); m 7,38 ppm (6H); m 5,05 ppm (1H); m 4,99 ppm (1H); m 3,98 ppm (1H); m 2,37 ppm (1H); s 2,08 ppm (3H); s 1,80 ppm (3H); m 1,70 ppm (3H); 1,58 ppm (1H); s 1,09 ppm (9H); d 1,02 ppm [7]Hz (3H)

### (1S,3S)-5-[[(1,1-Dimethylethyl)diphenylsilyl]oxy]-4-methyl-1,3-cyclohexandiol 17

469 mg (1 mmol) 16 werden in 10 ml THF gelöst und bei 0°C mit 3,4 ml Methylmagnesiumbromidlösung (3M in THF) versetzt. Nach 3 Std. bei dieser Temperatur wird mit Ammoniumchloridlösung hydrolysiert, mit Essigester extrahiert und über Natriumsulfat getrocknet. Das Solvens wird entfernt und der Rückstand an Kieselgel (Laufmittel: EE:H = 8:2) gereinigt, wobei man 231 mg (0,6 mmol) der Titelverbindung **17** erhält.
¹H-NMR (300 MHz, CDCl₃): δ= m 7,69 ppm (4H); m 7,42 ppm (6H); m 4,41 ppm (1H); m 4,09 ppm (1H); m 3,89 ppm (1H); m 2,32 ppm (1H); m 2,00 ppm (1H); m 1,58 ppm (1H); m 1,42 ppm (1H); 1,28 ppm (1H); s 1,09 ppm (9H); d 1,09 ppm [7]Hz (3H)

### (1S,5S)-1,5-Bis(1-ethoxyethoxy)-3-[[(1,1-dimethylethyl)diphenylsilyl]oxy]-2-methylcyclohexan 18

Man rührt 5,11 g (13,3 mmol) **17** mit 2,96 g (41 mmol) Ethylvinylether und 327 mg (1,3 mmol) Pyridinium-p-toluolsulfonat in 80 ml Dichlormethan bei Raumtemperatur für 3 Stunden. Anschließend wird mit Natriumchloridlösung gewaschen über Natriumsulfat getrocknet und eingeengt. Der Rückstand wird an Kieselgel (Laufmittel: EE:H = 1:1) chromatographiert, wobei 6,48 g (12,14 mmol) der Titelverbindung **18** anfallen.

### (3S,5R)-3,5-Bis(1-ethoxyethoxy)-2-methylcyclohexanol 19

6,48 g (12,14 mmol) **18** und 6,31 g (20 mmol) Tetrabutylammoniumfluorid (Hydrat) in 150 ml THF werden über Nacht bei Raumtemperatur gerührt. Anschließend hydrolysiert man mit Natriumchloridlösung, extrahiert mit Essigester, wäscht mit Natriumchloridlösung und trocknet über Natriumsulfat. Nach dem Einengen chromatographiert man den Rückstand an Kieselgel (Laufmittel: EE:H = 8:2), wobei man 2,41 g (8,3 mmol) der Titelverbindung **19** erhält.
¹H-NMR (300 MHz, CDCl₃): δ= m 4,78 ppm (2H); m 4,12 ppm (2H); m 3,89 ppm (1H); m 3,68 ppm (2H); m 3,49 ppm (2H); m 2,30 ppm (1H); m 1,70 ppm (1H); m 1,43 ppm (3H); d 1,34 ppm [7]Hz (6H); t 1,21 ppm [7]Hz (6H); d 1,09 ppm [7]Hz (3H)

### (3S,5S)-3,5-Bis(1-ethoxyethoxy)-2-methylcyclohexanon 20

Analog Beispiel 9 werden 2,4 g (8,3 mmol) 19 umgesetzt, wobei 2,17 g (7,5 mmol) der Titelverbindung 20 als farbloses Öl anfallen.
¹H-NMR (300 MHz, CDCl₃): δ= m 4,74 ppm (2H); m 4,18 ppm (2H); m 3,60 ppm (2H); m 3,48 ppm (2H); m 2,81 ppm (1H); m 2,42 ppm (3H); m 1,83 ppm (1H); d 1,38 ppm [7]Hz (6H); t 1,20 ppm [7]Hz (6H); d 1,09 ppm [7]Hz (3H)

### (3S,5S)-3,5-Bis(1-ethoxyethoxy)-1-ethinyl-2-methylcyclohexanol 21

Man setzt 2,00 g (6,94 mmol) **20** analog Beispiel **10** um und erhält 1,87 g (5,96 mmol) der Titelverbindung **21** als farbloses Öl.

### (3S,5S)-3,5-Bis(1-ethoxyethoxy)-1-ethinyl-2-methyl-1-cyclohexen 22

Analog Beispiel **11** setzt man 1,87 g (5,96 mmol) **21** um und erhält 1,39 g (4,67 mmol) der Titelverbindung **22** als gelbliches Öl.
¹H-NMR (300 MHz, CDCl₃): m 4,80 ppm (2H); m 4,00 ppm (2H); m 3,63 ppm (2H); m 3,49 ppm (2H); d 3,09 ppm [4]Hz (1H); m 2,54 ppm (1H); m 2,14 ppm (3H); s 2,00 ppm (3H); s 1,95 ppm (3H); d 1,38 ppm [7]Hz (6H); t 1,20 ppm [7]Hz (6H)

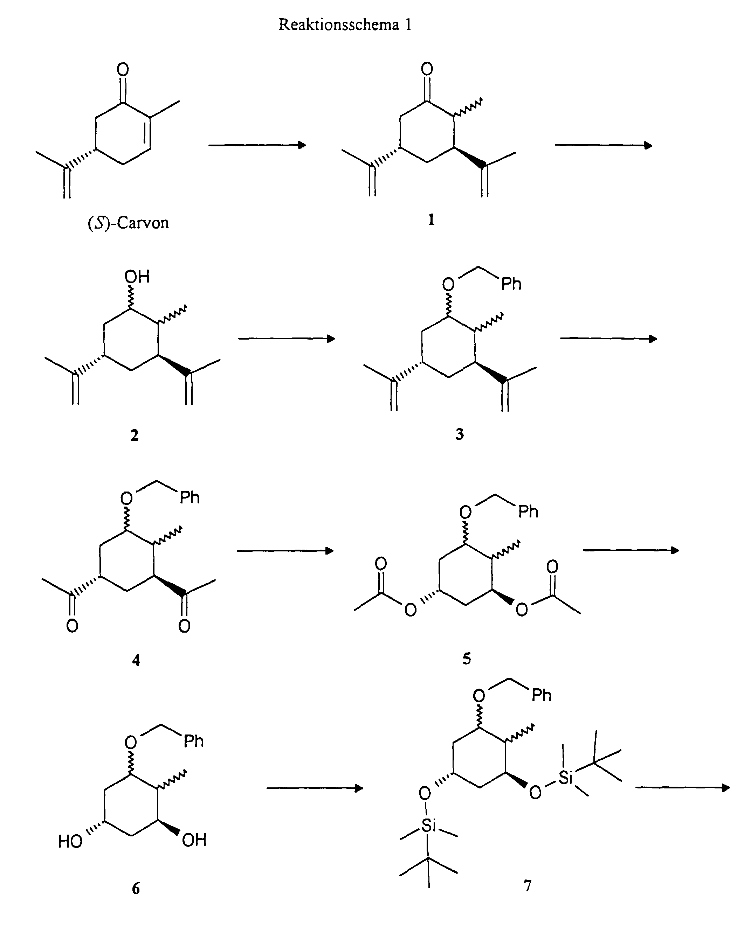

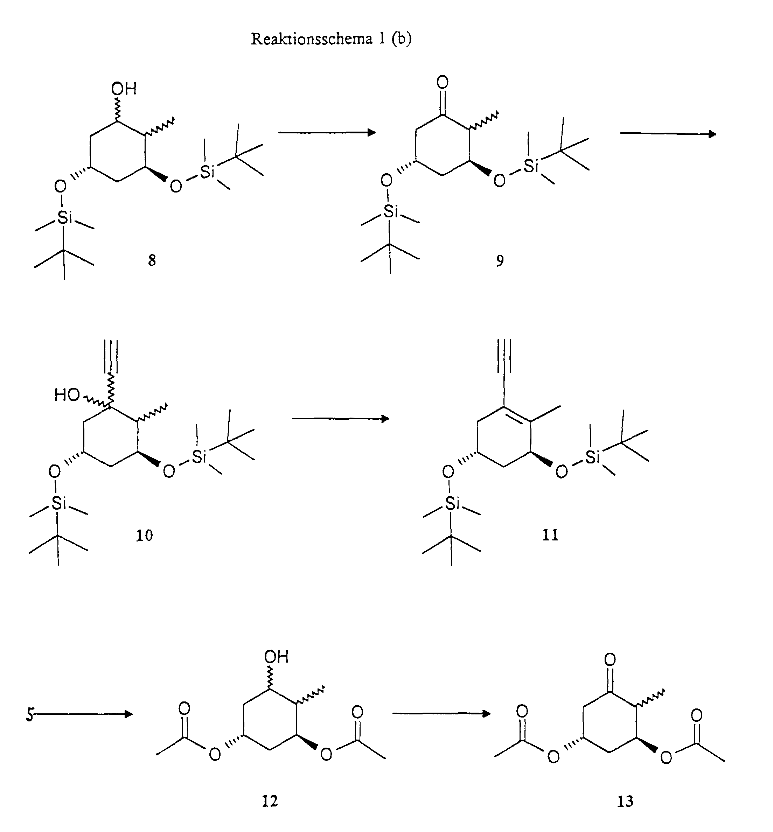

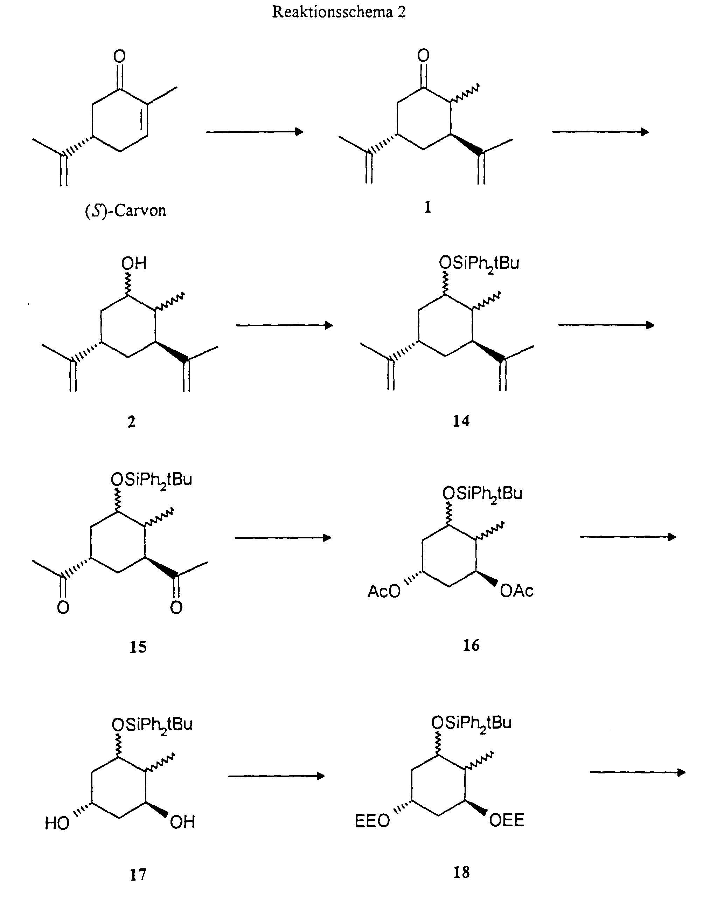

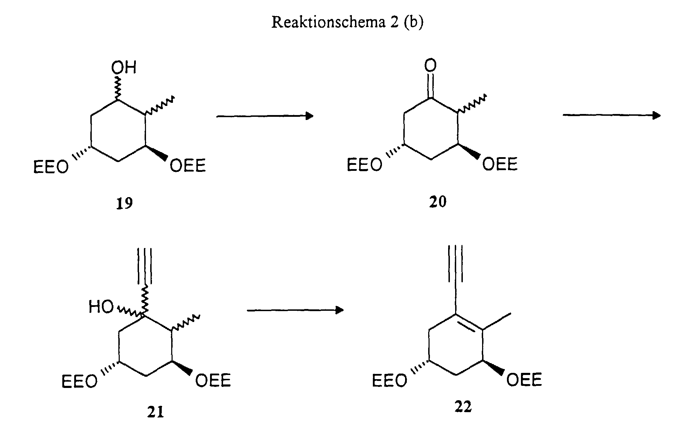

## Patentansprüche

1. Cyclohexanon-Derivate der allgemeinen Formel **I**, 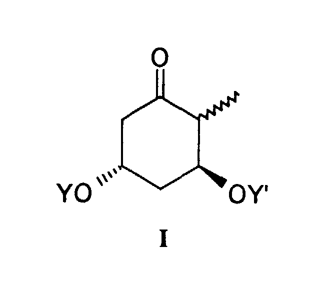 worin Y und Y' je ein Wasserstoffatom, eine Alkanoylgruppe mit 1 bis 9 Kohlenstoffatomen oder eine Aroylgruppe oder je eine alkyl- oder aryl- oder gemischt aryl-alkylsubstituierte Silylgruppe oder eine andere gängige Hydroxylschutzgruppe bedeuten.

2. Cyclohexanon-Derivate nach Anspruch 1, worin Y und Y' Acetyl, Propionyl, Pivaloyl oder Benzoyl oder Trimethylsilyl (TMS), Triethylsilyl (TES), *tert.*-Butyldimetylsilyl (TBDMS), *tert.*-Butyldiphenylsilyl (TBDPS) oder Triisopropylsilyl (TIPS) oder Methoxymethyl (MOM), Methoxyethoxymethyl (MEM), Ethoxyethyl (EE), Trimethylsilylethoxymethyl (SEM), Tetrahydrofuranyl (THF) oder Tetrahydropyranyl (THP) bedeuten und gleich oder verschieden sein können.

3. Cyclohexanon-Derivate nach Anspruch 1, nämlich
(3*S*,5*S*)-3,5-Bis[[dimethyl(1,1-dimethylethyl)silyl]oxy]-2-methylcyclohexanon,
(3*S*,5*S*)-3,5-Bis[[(1,1-dimethylethyl)diphenylsilyl]oxy]-2-methylcyclohexanon,
(3*S*,5*S*)-3,5-Bis[(triethylsilyl)oxy]-2-methylcyclohexanon,
(3*S*,5*S*)-3,5-Bis(acetyloxy)-2-methylcyclohexanon,
(3*S*,5*S*)-3,5-Bis(2,2-dimethyl-1-oxopropoxy)-2-methylcyclohexanon,
(3*S*,5*S*)-3,5-Bis(benzoyloxy)-2-methylcyclohexanon,
(3*S*,5*S*)-3,5-Bis(1-ethoxyethoxy)-2-methylcyclohexanon.

4. Verfahren zur Herstellung der Cyclohexanon-Derivate der allgemeinen Formel **I** nach Anspruch 1, durch Umsetzung von (*S*)-Carvon mit einer kupferhaltigen metallorganischen Verbindung in die Verbindung **VIII**, 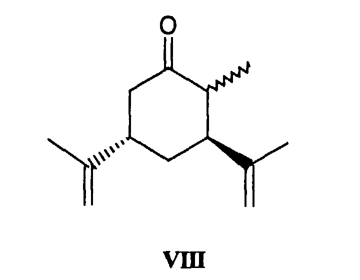 Umsetzung der Verbindung **VIII** in die Verbindung der allgemeinen Formel **IX** 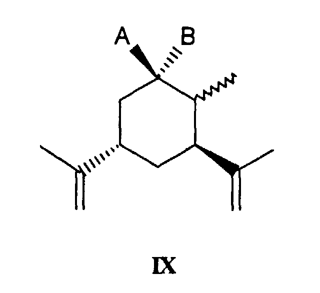 anschließende oxidative Spaltung (z.B. Ozonolyse) der Vinylgruppen zu den korrespondierenden Methylketonen der allgemeinen Formel **X**, 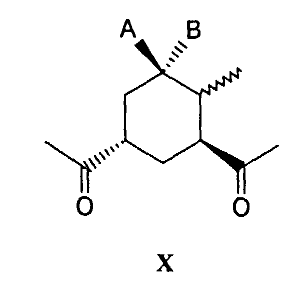 Überführung der Verbindungen der allgemeinen Formel **X** durch Baeyer-Villiger-Oxidation in das Diacetat der allgemeinen Formel **XI** 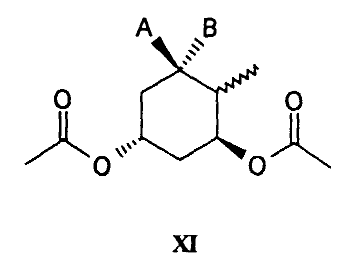 Verseifung der Acetatgruppen und Überführung in Verbindungen der allgemeinen Formel **XII,** 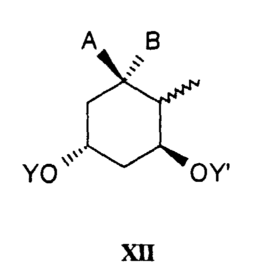 worin Y und Y' die in Anspruch 1 und 2 genannten Bedeutungen haben, und in den allgemeinen Formeln **IX**, **X**, **XI** und **XII** A und B gemeinsam eine freie oder geschützte Carbonylgruppe bedeuten, wobei die Schutzgruppen ausgewählt sind aus Dialkoxyketal, 1,3-Dioxan, 1,3-Dioxolan, Dialkylhydrazon, Tosylhydrazon, Oxim, Alkyl- oder Benzyloximether oder A oder B ein Wasserstoffatom und die entsprechende andere Gruppe B oder A eine Hydroxygruppe, welche frei oder geschützt sein kann, wobei die Schutzgruppen ausgewählt sind aus Benzylether, p-Methoxybenzylether, o-, m-, p-Nitrobenzylether, TBDMS-Ether, TIPS-Ether, TBDPS-Ether, TES-Ether, MOM-Ether, MEM-Ether, SEM-Ether, EE-Ether, THP-Ether und THF-Ether, und anschließende Spaltung der Ketal- oder Hydrazon-Gruppe oder selektive Spaltung der Alkoholschutzgruppe A oder B und Oxidation.

5. Verbindungen der allgemeinen Formel **IX** 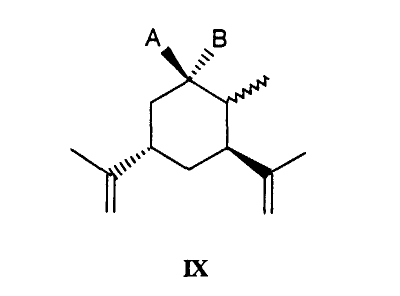 worin A und B eine geschützte Carbonylgruppe bedeuten, wobei die Schutzgruppen ausgewählt sind aus Dialkoxyketal, 1,3-Dioxan, 1,3-Dioxolan, Dialkylhydrazon, Tosylhydrazon, Oxim, Alkyl- oder Benzyloximether oder A oder B ein Wasserstoffatom und die entsprechende andere Gruppe B oder A eine Hydroxygruppe, welche frei oder geschützt sein kann, wobei die Schutzgruppen ausgewählt sind aus Benzylether, p-Methoxybenzylether, o-, m-, p-Nitrobenzylether, TBDMS-Ether, TIPS-Ether, TBDPS-Ether, TES-Ether, MOM-Ether, MEM-Ether, SEM-Ether, EE-Ether, THP-Ether und THF-Ether.

6. Verbindung gemäß Anspruch 5, nämlich
(1*S*,5*S*)-1,5-Bis(1-methylethenyl)-2-methyl-3-(phenylmethoxy)cyclohexan,
(*E*)-(3*S*,5*S*)-3,5-Bis(1-methylethenyl)-2-methylcyclohexanonoxim,
(*Z*)-(3*S*,5*S*)-3,5-Bis(1-methylethenyl)-2-methylcyclohexanonoxim,
(*E*)-(3*S*,5*S*)-3,5-Bis(1-methylethenyl)-2-methylcyclohexanon-O-methyloxim,
(*Z*)-(3*S*,5*S*)-3,5-Bis(1-methylethenyl)-2-methylcyclohexanon-O-methyloxim,
(1*S*,5*S*)-1,5-Bis(1-methylethenyl)-3-[[(1,1-dimethylethyl)diphenylsilyl]oxy]-2-methylcyclohexan.

7. Verbindungen der allgemeinen Formel **X**, 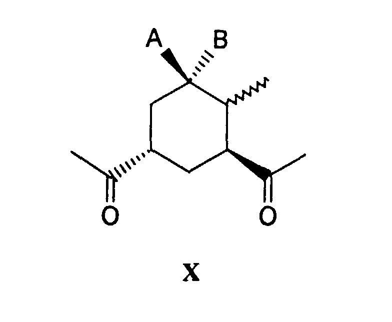 worin A und B gemeinsam eine freie oder geschützte Carbonylgruppe bedeuten, wobei die Schutzgruppen ausgewählt sind aus Dialkoxyketal, 1,3-Dioxan, 1,3-Dioxolan, Dialkylhydrazon, Tosylhydrazon, Oxim, Alkyl- oder Benzyloximether oder A oder B ein Wasserstoffatom und die entsprechende andere Gruppe B oder A eine Hydroxygruppe, welche frei oder geschützt sein kann, wobei die Schutzgruppen ausgewählt sind aus Benzylether, p-Methoxybenzylether, o-, m-, p- Nitrobenzylether, TBDMS-Ether, TIPS-Ether, TBDPS-Ether, TES-Ether, MOM-Ether, MEM-Ether, SEM-Ether, EE-Ether, THP-Ether und THF-Ether.

8. Verbindung gemäß Anspruch 7, nämlich
(1*S*,3*S*)-1,1'-[4-Methyl-5-(phenylmethoxy)-1,3-cyclohexandiyl]bis[ethanon],
(*E*)-(1*S*,3*S*)-1,1'-[5-(Hydroxyimino)-4-methyl-1,3-cyclohexandiyl]bis[ethanon],
(*Z*)-(1*S*,3*S*)-1,1'-[5-(Hydroxyimino)-4-methyl-1,3-cyclohexandiyl]bis[ethanon],
(*E*)-(1*S*,3*S*)-1,1'-[5-(Methoxyimino)-4-methyl-1,3-cyclohexandiyl]bis[ethanon],
(*Z*)-(1*S*,3*S*)-1,1'-[5-(Methoxyimino)-4-methyl-1,3-cyclohexandiyl]bis[ethanon],
(1*S*,3*S*)-1,1'-[5-[[(1,1-Dimethylethyl)diphenylsilyl]oxy]-4-methyl-1,3-cyclohexandiyl]bis[ethanon].

9. Verbindungen der allgemeinen Formel **XI**, 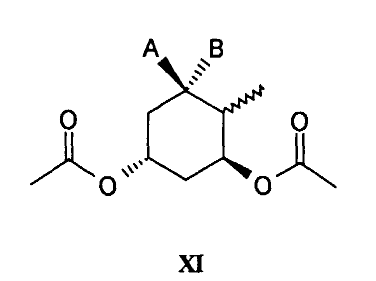 worin A und B gemeinsam eine freie oder geschützte Carbonylgruppe bedeuten, wobei die Schutzgruppen ausgewählt sind aus Dialkoxyketal, 1,3-Dioxan, 1,3-Dioxolan, Dialkylhydrazon, Tosylhydrazon, Oxim, Alkyl- oder Benzyloximether oder A oder B ein Wasserstoffatom und die entsprechende andere Gruppe B oder A eine Hydroxygruppe, welche frei oder geschützt sein kann, wobei die Schutzgruppen ausgewählt sind aus Benzylether, p-Methoxybenzylether, o-, m-, p- Nitrobenzylether, TBDMS-Ether, TIPS-Ether, TBDPS-Ether, TES-Ether, MOM-Ether, MEM-Ether, SEM-Ether, EE-Ether, THP-Ether und THF-Ether.

10. Verbindung gemäß Anspruch 9, nämlich
(1*S*,5*R*)-1,5-Bis(acetyloxy)-2-methyl-3-(phenylmethoxy)cyclohexan,
(3*S*,5*R*)-3,5-Bis(acetyloxy)-2-methylcyclohexanol,
(*E*)-(3*S*,5*R*)-3,5-Bis(acetyloxy)-2-methylcyclohexanonoxim,
(*Z*)-(3*S*,5*R*)-3,5-Bis(acetyloxy)-2-methylcyclohexanonoxim,
(*E*)-(3*S*,5*R*)-3,5-Bis(acetyloxy)-2-methylcyclohexanon-O-methyloxim,
(*Z*)-(3*S*,5*R*)-3,5-Bis(acetyloxy)-2-methylcyclohexanon-O-methyloxim,
(1*S*,5*S*)-1,5-Bis(acetyloxy)-3-[[(1,1-dimethylethyl)diphenylsilyl]oxy]-2-methylcyclohexan.

11. Verbindungen der allgemeinen Formel **XII,** 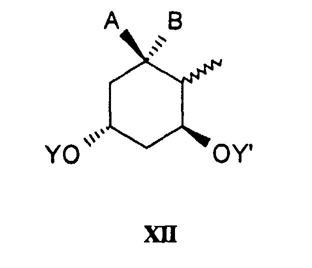 worin Y und Y' je ein Wasserstoffatom, eine Alkanoylgruppe mit 1 bis 9 Kohlenstoffatomen oder eine Aroylgruppe oder je eine alkyl- oder aryl- oder gemischt aryl-alkylsubstituierte Silylgruppe oder eine andere gängige Schutzgruppe bedeuten,
und A und B gemeinsam eine freie oder geschützte Carbonylgruppe bedeuten, wobei die Schutzgruppen ausgewählt sind aus Dialkoxyketal, 1,3-Dioxan, 1,3-Dioxolan, Dialkylhydrazon, Tosylhydrazon, Oxim, Alkyl- oder Benzyloximether oder A oder B ein Wasserstoffatom und die entsprechende andere Gruppe B oder A eine Hydroxygruppe, welche frei oder geschützt sein kann, wobei die Schutzgruppen ausgewählt sind aus Benzylether, p-Methoxybenzylether, o-, m-, p- Nitrobenzylether, TBDMS-Ether, TIPS-Ether, TBDPS-Ether, TES-Ether, MOM-Ether, MEM-Ether, SEM-Ether, EE-Ether, THP-Ether und THF-Ether.

12. Verbindung gemäß Anspruch 11, nämlich
(1*S*,3*S*)-4-Methyl-5-(phenylmethoxy)-1,3-cyclohexandiol,
(1*S*,5*R*)-1,5-Bis[[dimethyl(1,1-dimethylethyl)silyl]oxy]2-methyl-3-(phenylmethoxy)cyclohexan,
(3*S*,5*R*)-3,5-Bis[[dimethyl(1,1-dimethylethyl)silyl]oxy]-2-methylcyclohexanol,
(3*S*,5*R*)-3,5-Bis(acetyloxy)-2-methylcyclohexanol,
(*E*)-(3*S*,5*R*)-3,5-Dihydroxy-2-methylcyclobexanonoxim,
(*Z*)-(3*S*,5*R*)-3,5-Dihydroxy-2-methylcyclohexanonoxim,
(*E*)-(3*S*,5*R*)-3,5-Dihydroxy-2-methylcyclohexanon-O-methyloxim,
(*Z*)-(3S*,*5*R*)-3,5-Dihydroxy-2-methylcyclohexanon-O-methyloxim,
(*E*)-(3*S*,5*R*)-3,5-Bis[[dimethyl(1,1-dimethylethyl)silyl]oxy]-2-methylcyclohexanonoxim,
(*Z*)-(3*S*,5*R*)-3,5-Bis[[dimethyl(1,1-dimethylethyl)silyl]oxy]-2-methylcyclohexanonoxim,
(*E*)-(3*S*,5*R*)-3,5-Bis[[dimethyl(1,1-dimethylethyl)silyl]oxy]-2-methylcyclohexanon-O-methyloxim,
(*Z*)-(3*S*,5*R*)-3,5-Bis[[dimethyl(1,1-dimethylethyl)silyl]oxy]-2-methylcyclohexanon-O-methyloxim,
(1*S*, 5*S*)-1,5-Bis(acetyloxy)-3-[[(1,1-dimethylethyl)diphenylsilyl]oxy]-2-methylcyclohexan,
(1*S*,3*S*)-5-[[(1,1-Dimethylethyl)diphenylsilyl]oxy]-4-methyl-1,3-cyclohexandiol,
(1*S*,5*S*)-1,5-Bis(1-ethoxyethoxy)-3-[[(1,1-dimethylethyl)diphenylsilyl]oxy]-2-methylcyclohexan,
(3*S*,5*R*)-3,5-Bis(1-ethoxyethoxy)-2-methylcyclohexanol.

## Claims

1. Cyclohexanone derivatives of the general formula I, 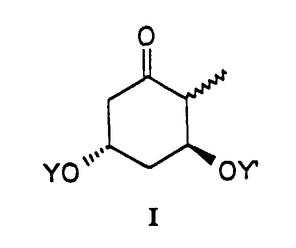 in which Y and Y' are each a hydrogen atom, an alkanoyl group having from 1 to 9 carbon atoms or an aroyl group or are each an alkyl- or aryl- or mixed aryl-alkylsubstituted silyl group or another common hydroxylprotecting group.

2. Cyclohexanone derivatives according to Claim 1, in which Y and Y' are acetyl, propionyl, pivaloyl or benzoyl or trimethylsilyl (TMS), triethylsilyl (TES), tert-butyldimethylsilyl (TBDMS), tert-butyldiphenylsilyl (TBDPS) or triisopropylsilyl (TIPS) or methoxymethyl (MOM), methoxyethoxymethyl (MEM), ethoxyethyl (EE), trimethylsilylethoxymethyl (SEM), tetrahydrofuranyl (THF) or tetrahydropyranyl (THP), and can be identical or different.

3. Cyclohexanone derivatives according to Claim 1, namely
(3*S*,5*S*)-3,5-bis[[dimethyl(1,1-dimethylethyl)silyl]oxy]-2-methylcyclohexanone,
(3*S*,5*S*)-3,5-bis[[(1,1-dimethylethyl)diphenylsilyl]oxy]2-methylcyclohexanone,
(3*S*,5*S*)-3,5-bis[(triethylsilyl)oxy]-2-methylcyclohexanone,
(3*S*,5*S*)-3,5-bis(acetyloxy)-2-methylcyclohexanone,
(3*S*,5*S*)-3,5-bis(2,2-dimethyl-1-oxopropoxy)-2-methylcyclohexanone,
(3*S*,5*S*)-3,5-bis(benzoyloxy)-2-methylcyclohexanone,
(3*S*,5*S*)-3,5-bis(1-ethoxyethoxy)-2-methylcyclohexanone.

4. Process for the preparation of the cyclohexanone derivatives of the general formula I according to Claim 1, by reaction of (*S*)-carvone with a copper-containing organometallic compound to give the compound VIII, 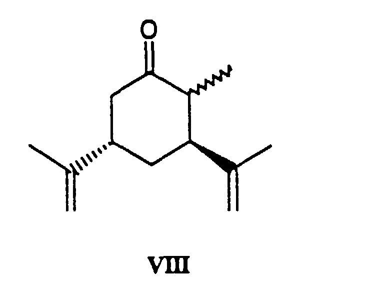 reaction of the compound VIII to give the compound of the general formula IX, 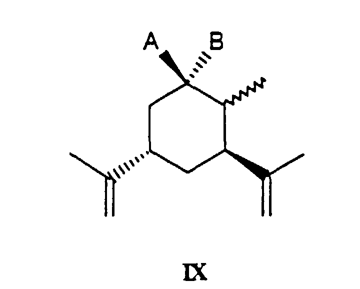 subsequent oxidative cleavage (e.g. ozonolysis) of the vinyl groups to give the corresponding methyl ketones of the general formula X, 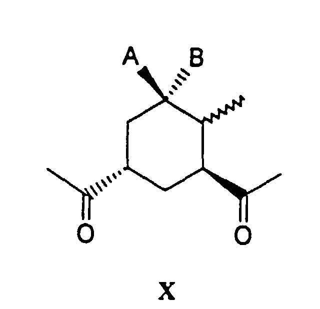 conversion of the compounds of the general formula X by Baeyer-Villiger oxidation into the diacetate of the general formula XI, 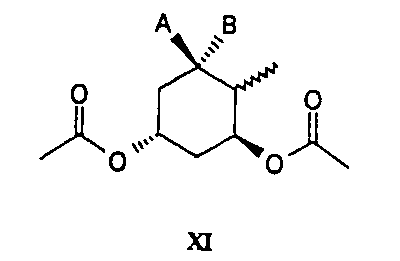 hydrolysis of the acetate groups and conversion into compounds of the general formula XII, 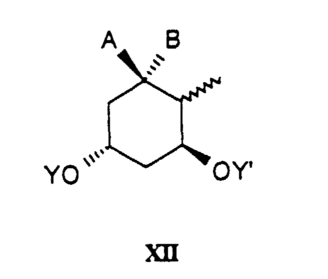 in which Y and Y' are as defined in Claim 1 and 2, and in the general formulae IX, X, XI and XII, A and B are jointly a free or protected carbonyl group, the protected groups being chosen from dialkoxyketal, 1,3-dioxane, 1,3-dioxolane, dialkylhydrazone, tosylhydrazone, oxime, alkyl- or benzyloxime ether, or A or B is a hydrogen atom and the corresponding other group B or A is a hydroxyl group, which can be free or protected, the protective groups being chosen from benzyl ether, p-methoxybenzyl ether, o-, m-, p-nitrobenzyl ether, TBDMS ether, TIPS ether, TBDPS ether, TES ether, MOM ether, MEM ether, SEM ether, EE ether, THP ether and THF ether, and subsequent cleavage of the ketal or hydrazone group or selective cleavage of the alcohol-protecting group A or B and oxidation.

5. Compounds of the general formula IX, 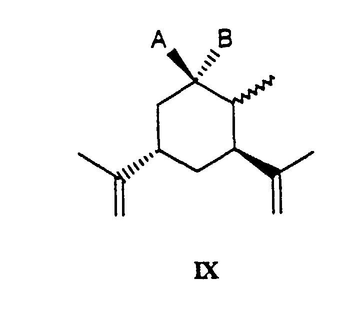 in which A and B are a protected carbonyl group, the protective groups being chosen from dialkoxyketal, 1,3-dioxane, 1,3-dioxolane, dialkylhydrazone, tosylhydrazone, oxime, alkyl- or benzyloxime ether, or A or B is a hydrogen atom and the corresponding other group B or A is a hydroxyl group, which can be free or protected, the protective groups being chosen from benzyl ether, p-methoxybenzyl ether, o-, m-, p-nitrobenzyl ether, TBDMS ether, TIPS ether, TBDPS ether, TES ether, MOM ether, MEM ether, SEM ether, EE ether, THP ether and THF ether.

6. Compound according to Claim 5, namely
(1*S*,5*S*)-1,5-bis(1-methylethenyl)-2-methyl-3-(phenylmethoxy)cyclohexane,
(*E*)-(3*S*,5*S*)-3,5-bis(1-methylethenyl)-2-methylcyclohexanone oxime,
(*Z*)-(3*S*,5*S*)-3,5-bis(1-methylethenyl)-2-methylcyclohexanone oxime,
(*E*)-(3*S*,5*S*)-3,5-bis(1-methylethenyl)-2-methylcyclohexanone O-methyloxime,
(*Z*)-(3*S*,5*S*)-3,5-bis(1-methylethenyl)-2-methylcyclohexanone O-methyloxime,
(1*S*,5*S*)-1,5-bis(1-methylethenyl)-3-[[(1,1-dimethylethyl)diphenylsilyl]oxy]-2-methylcyclohexane.

7. Compounds of the general formula X, 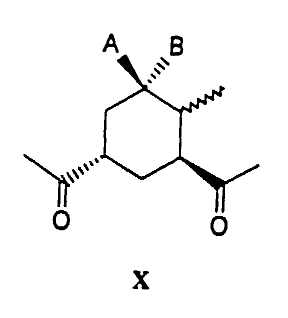 in which A and B are jointly a free or protected carbonyl group, the protective groups being chosen from dialkoxyketal, 1,3-dioxane, 1,3-dioxolane, dialkylhydrazone, tosylhydrazone, oxime, alkyl- or benzyloxime ether, or A or B is a hydrogen atom and the corresponding other group B or A is a hydroxyl group, which can be free or protected, the protective groups being chosen from benzyl ether, p-methoxybenzyl ether, o-, m-, p-nitrobenzyl ether, TBDMS ether, TIPS ether, TBDPS ether, TES ether, MOM ether, MEM ether, SEM ether, EE ether, THP ether and THF ether.

8. Compound according to Claim 7, namely
(1*S*,3*S*)-1,1'-[4-methyl-5-(phenylmethoxy)-1,3-cyclohexanediyl]bis[ethanone],
(*E*)-(1*S*,3*S*)-1,1'-[5-(hydroxyimino)-4-methyl-1,3-cyclohexanediyl]bis[ethanone],
(*Z*)-(1*S*,3*S*)-1,1'-[5-(hydroxyimino)-4-methyl-1,3-cyclohexanediyl]bis[ethanone],
(*E*)-(1*S*,3*S*)-1,1'-[5-(methoxyimino)-4-methyl-1,3-cyclohexanediyl]bis[ethanone],
(*Z*)-(1*S*,3*S*)-1,1'-[5-(methoxyimino)-4-methyl-1,3-cyclohexanediyl]bis[ethanone],
(1*S*,3*S*)-1,1'-[5-[[(1,1-dimethylethyl)diphenylsilyl]oxy]-4-methyl-1,3-cyclohexanediyl]bis[ethanone].

9. Compounds of the general formula XI, 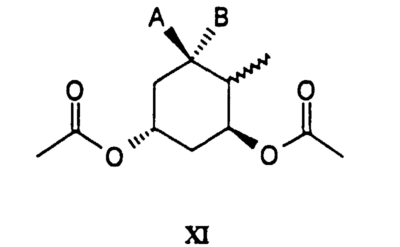 in which A and B are jointly a free or protected carbonyl group, the protective groups being chosen from dialkoxyketal, 1,3-dioxane, 1,3-dioxolane, dialkylhydrazone, tosylhydrazone, oxime, alkyl- or benzyloxime ether, or A or B is a hydrogen atom and the corresponding other group B or A is a hydroxyl group, which can be free or protected, the protective groups being chosen from benzyl ether, p-methoxybenzyl ether, o-, m-, p-nitrobenzyl ether, TBDMS ether, TIPS ether, TBDPS ether, TES ether, MOM ether, MEM ether, SEM ether, EE ether, THP ether and THF ether.

10. Compound according to Claim 9, namely
(1*S*,5*R*)-1,5-bis(acetyloxy)-2-methyl-3-(phenylmethoxy)cyclohexane,
(3*S*,5*R*)-3,5-bis (acetyloxy)-2-methylcyclohexanol,
(*E*)-(3*S*,5*R*)-3,5-bis (acetyloxy)-2-methylcyclohexanone oxime,
(*Z*)-(3*S*,5*R*)-3,5-bis(acetyloxy)-2-methylcyclohexanone oxime,
(*E*) - (3*S*,5*R*)-3,5-bis(acetyloxy)-2-methylcyclohexanone O-methyloxime,
(*Z*)-(3*S*,5*R*)-3,5-bis(acetyloxy)-2-methylcyclohexanone O-methyloxime,
(1*S*,5*S*)-1,5-bis(acetyloxy)-3-[[(1,1-dimethylethyl)diphenylsilyl]oxy]-2-methylcyclohexane.

11. Compounds of the general formula XII, 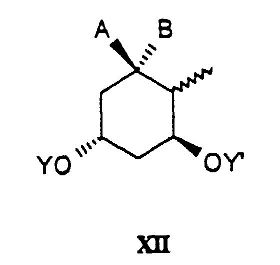 in which Y and Y' are each a hydrogen atom, an alkanoyl group having from 1 to 9 carbon atoms or an aroyl group or are each an alkyl- or aryl- or mixed aryl-alkylsubstituted silyl group or another common protective group,
and A and B are jointly a free or protected carbonyl group, the protective groups being chosen from dialkoxyketal, 1,3-dioxane, 1,3-dioxolane, dialkylhydrazone, tosylhydrazone, oxime, alkyl- or benzyloxime ether, or A or B is a hydrogen atom and the corresponding other group B or A is a hydroxyl group, which can be free or protected, the protective groups being chosen from benzyl ether, p-methoxybenzyl ether, o-, m-, p-nitrobenzyl ether, TBDMS ether, TIPS ether, TBDPS ether, TES ether, MOM ether, MEM ether, SEM ether, EE ether, THP ether and THF ether.

12. Compound according to Claim 11, namely
(1*S*,3*S*)-4-methyl-5-(phenylmethoxy)-1,3-cyclohexanediol,
(1*S*,5*R*)-1,5-bis[[dimethyl(1,1-dimethylethyl)silyl]oxy]-2-methyl-3-(phenylmethoxy)cyclohexane,
(3*S*,5*R*)-3,5-bis[[dimethyl(1,1-dimethylethyl)silyl]oxy]-2-methylcyclohexanol,
(3*S*,5*R*)-3,5-bis (acetyloxy)-2-methylcyclohexanol,
(*E*)-(3*S*,5*R*)-3,5-dihydroxy-2-methylcyclohexanone oxime,
(*Z*)-(3*S*,5*R*)-3,5-dihydroxy-2-methylcyclohexanone oxime,
(*E*)-(3*S*,5*R*)-3,5-dihydroxy-2-methylcyclohexanone O-methyloxime,
(*Z*)-(3*S*,5*R*)-3,5-dihydroxy-2-methylcyclohexanone O-methyloxime,
(*E*)-(3*S*,5*R*)-3,5-bis[[dimethyl(1,1-dimethylethyl)silyl]oxy]-2-methylcyclohexanone oxime,
(*Z*)-(3*S*,5*R*)-3,5-bis[[dimethyl(1,1-dimethylethyl)silyl]oxy]-2-methylcyclohexanone oxime,
(*E*)-(3*S*,5*R*)-3,5-bis[[dimethyl(1,1-dimethylethyl)silyl]oxy]-2-methylcyclohexanone O-methyloxime,
(*Z*)-(3*S*,5*R*)-3,5-bis[[dimethyl(1,1-dimethylethyl)silyl]oxy]-2-methylcyclohexanone O-methyloxime,
(1*S*,5*S*)-1,5-bis (acetyloxy)-3-[[(1,1-dimethylethyl)diphenylsilyl]oxy] -2-methylcyclohexane,
(1*S*,3*S*)-5-[[(1,1-dimethylethyl)diphenylsilyl]oxy]-4-methyl-1,3-cyclohexanediol,
(1*S*,5*S*)-1,5-bis(1-ethoxyethoxy)-3-[[(1,1-dimethylethyl)diphenylsilyl]oxy]-2-methylcyclohexane,
(3*S*,5*R*)-3,5-bis (l-ethoxyethoxy)-2-methylcyclohexanol.

## Revendications

1. Dérivés de cyclohexanone de formule générale I, 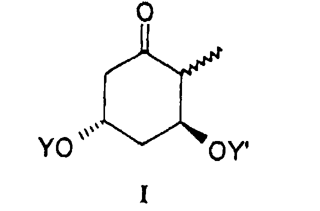 dans laquelle Y et Y' signifient chacun un atome d'hydrogène, un groupement alcanoyle ayant 1 à 9 atomes de carbone ou un groupement aroyle ou chacun un groupement alkyle ou aryle ou silyle substitué par un mélange aryle/alkyle ou un autre groupement protecteur hydroxyle courant.

2. Dérivés de cyclohexanone suivant la revendication 1, dans lesquels Y et Y' signifient un acétyle, un propionyle, un pivaloyle ou un benzoyle ou un triméthylsilyle (TMS), un triéthylsilyle (TES), un t-butyldiméthylsilyle (TBDMS), un t-butyldiphénylsilyle (TBDPS) ou un triisopropylsilyle (TIPS) ou un méthoxyméthyle (MOM), un méthoxyéthoxyméthyle (MEM), un éthoxyéthyle (EE), un triméthylsilyléthoxyméthyle (SEM), un tétrahydrofurannyle (THF) ou un tétrahydropyrannyle (THP) et peuvent être identiques ou différents.

3. Dérivés de cyclohexanone suivant la revendication 1, à savoir
(3*S*,5*S*)-3,5-bis[[diméthyl(1,1-diméthyléthyl)silyl]oxy]-2-méthylcyclohexanone;
(3*S*,5*S*)-3,5-bis[[(1,1-diméthyléthyl)diphénylsilyl]oxy]-2-méthylcyclohexanone;
(3*S*,5*S*)-3,5-bis[(triéthylsilyl)oxy]-2-méthylcyclohexanone;
(3*S*,5*S*)-3,5-bis(acétyloxy)-2-méthylcyclohexanone;
(3*S*,5*S*)-3,5-bis(2,2-diméthyl-1-oxopropoxy)-2-méthylcyclohexanone;
(3*S*,5*S*)-3,5-bis(benzoyloxy)-2-méthylcyclohexanone;
(3*S*,5*S*)-3,5-bis (1-éthoxyéthoxy)-2-méthylcyclohexanone.

4. Procédé de préparation des dérivés de cyclohexanone de formule générale I suivant la revendication 1, par transformation de (S)-carvone avec un composé organométallique contenant du cuivre en le composé VIII, 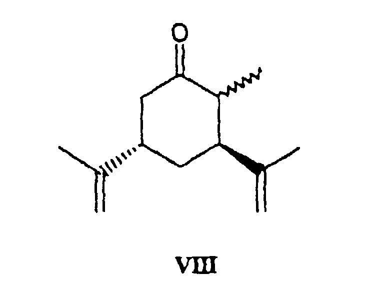 transformation du composé VIII en le composé de formule générale IX 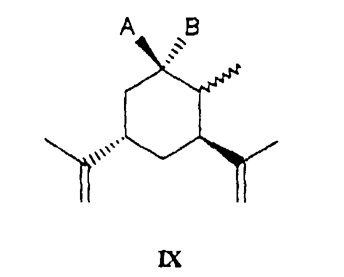 dissociation oxydative ultérieure (par exemple ozonolyse) des groupements vinyle en les méthylcétones correspondantes de formule générale X, 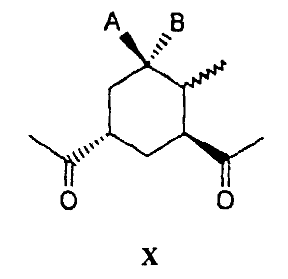 conversion des composés de formule générale X par oxydation de Baeyer-Villiger en le diacétate de formule générale XI 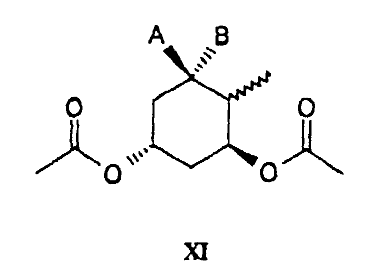 saponification des groupements acétate et conversion en composés de formule générale XII, 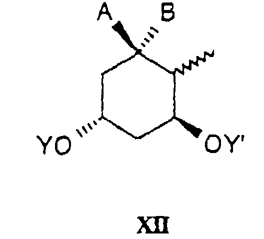 dans laquelle Y et Y' ont les significations données dans les revendications 1 et 2, et A et B signifient ensemble, dans les formules générales IX, X, XI et XII, un groupement carbonyle libre ou protégé, les groupements protecteurs étant sélectionnés parmi dialkoxycétal, 1,3-dioxanne, 1,3-dioxolanne, dialkylhydrazone, tosylhydrazone, oxime, oximéther d'alkyle ou de benzyle, ou A ou B peut être un atome d'hydrogène, et l'autre groupement correspondant B ou A peut être un groupement hydroxyle, lequel peut être libre ou protégé, les groupements protecteurs étant sélectionnés parmi benzyléther, p-méthoxybenzyléther, o-, m-, p-nitrobenzyléther, éther-TBDMS, éther-TIPS, éther-TBDPS, éther-TES, éther-MOM, éther-MEM, éther-SEM, éther-EE, éther-THP et éther-THF, et dissociation ultérieure du groupement cétal ou hydrazone ou dissociation sélective du groupement protecteur de l'alcool A ou B et oxydation.

5. Composés de formule générale IX 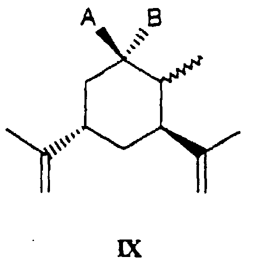 dans laquelle A et B signifient un groupement carbonyle protégé, les groupements protecteurs étant sélectionnés parmi dialkoxycétal, 1,3-dioxanne, 1,3-dioxolanne, dialkylhydrazone, tosylhydrazone, oxime, oximéther d'alkyle ou de benzyle ou A ou B peut être un atome d'hydrogène et l'autre groupement correspondant B ou A peut être un groupement hydroxyle, lequel peut être libre ou protégé, les groupements protecteurs étant sélectionnés parmi benzyléther, p-méthoxybenzyléther, o-, m-, p-nitrobenzyléther, éther-TBDMS, éther-TIPS, éther-TBDPS, éther-TES, éther-MOM, éther-MEM, éther-SEM, éther-EE, éther-THP et éther-THF.

6. Composé suivant la revendication 5, à savoir
(1*S*,5*S*)-1,5-bis(1-méthyléthényl)-2-méthyl-3-(phénylméthoxy)cyclohexane;
(*E*)-(3*S*,5*S*)-3,5-bis(1-méthyléthényl)-2-méthylcyclohexanonoxime;
(*Z*)- (3*S*,5*S*)-3,5-bis(1-méthyléthényl)-2-méthylcyclohexanonoxime;
(*E*)- (3*S*,5*S*)-3,5-bis(1-méthyléthényl)-2-méthylcyclohexanone-O-méthyloxime;
(*Z*)-(3*S*,5*S*)-3,5-bis(1-méthyléthényl)-2-méthylcyclohexanone-O-méthyloxime;
(1*S*,5*S*)-1,5-bis(1-méthyléthényl)-3-[[(1,1-diméthyléthyl)diphénylsilyl]oxy]-2-méthylcyclohexane.

7. Composés de formule générale X, 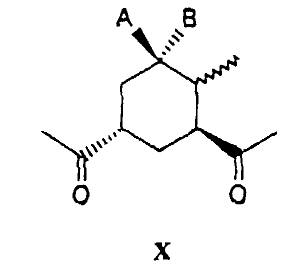 dans laquelle A et B signifient ensemble un groupement carbonyle libre ou protégé, les groupements protecteurs étant sélectionnés parmi dialkoxycétal, 1,3-dioxanne, 1,3-dioxolanne, dialkylhydrazone, tosylhydrazone, oxime, oximéther d'alkyle ou de benzyle ou A ou B peut être un atome d'hydrogène et l'autre groupement correspondant B ou A peut être un groupement hydroxyle, lequel peut être libre ou protégé, les groupements protecteurs étant sélectionnés parmi benzyléther, p-méthoxybenzyléther, o-, m-, p-nitrobenzyléther, éther-TBDMS, éther-TIPS, éther-TBDPS, éther-TES, éther-MOM, éther-MEM, éther-SEM, éther-EE, éther-THP et éther-THF.

8. Composé suivant la revendication 7, à savoir
(1*S*,3*S*)-1,1'-[4-méthyl-5-(phénylméthoxy)-1,3-cyclohexandiyl]bis[éthanone] ;
(*E*)-(1*S*,3*S*)-1,1'-[5-(hydroxyimino)-4-méthyl-1,3-cyclohexandiyl]bis[éthanone] ;
(*Z*)-(1*S*,3*S*)-1,1'-[5-(hydroxyimino)-4-méthyl-1,3-cyclohexandiyl]bis[éthanone] ;
(*E*)-(1*S*,3*S*)-1,1'-[5-(méthoxyimino)-4-méthyl-1,3-cyclohexandiyl]bis[éthanone] ;
(*Z*)-(1*S*,3*S*)-1,1'-[5-(méthoxyimino)-4-méthyl-1,3-cyclohexandiyl]bis[éthanone] ;
(1*S*,3*S*)-1,1'-[5-[[(1,1-diméthyléthyl)diphénylsilyl]oxy]-4-méthyl-1,3-cyclohexandiyl]bis[éthanone].

9. Composés de formule générale XI, 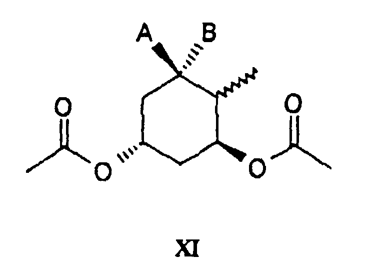 dans laquelle A et B signifient ensemble un groupement carbonyle libre ou protégé, les groupements protecteurs étant sélectionnés parmi dialkoxycétal, 1,3-dioxanne, 1,3-dioxalanne, dialkylhydrazone, tosylhydrazone, oxime, oximéther d'alkyle ou de benzyle ou A ou B peut être un atome d'hydrogène et l'autre groupe correspondant B ou A peut être un groupement hydroxyle, lequel peut être libre ou protégé, les groupements protecteurs étant sélectionnés parmi benzyléther, p-méthoxybenzyléther, o-, m-, p-nitro benzyléther, éther-TBDMS, éther-TIPS, éther-TBDPS, éther-TES, éther-MOM, éther-MEM, éther-SEM, éther-EE, éther-THP et éther-THF.

10. Composé suivant la revendication 9, à savoir
(1*S*,5*R*)-1,5-bis(acétyloxy)-2-méthyl-3-(phényl-méthoxy) cyclohexane;
(3*S*,5*R*)-3,5-bis(acétyloxy)-2-méthylcyclohexanol;
(*E*)-(3*S*,5*R*)-3,5-bis(acétyloxy)-2-méthyl-cyclohexanonoxime;
(*Z*)-(3*S*,5*R*)-3,5-bis(acétyloxy)-2-méthyl-cyclohexanonoxime;
(*E*)-(3*S*,5*R*)-3,5-bis(acétyloxy)-2-méthyl-cyclohexanone-O-méthyloxime;
(*Z*)-(3*S*,5*R*)-3,5-bis(acétyloxy)-2-méthyl-cyclohexanone-O-méthyloxime;
(1*S*,5*S*)-1,5-bis(acétyloxy)-3-[[(1,1-diméthyléthyl)diphénylsilyl]oxy]-2-méthylcyclohexane.

11. Composés de formule générale XII, 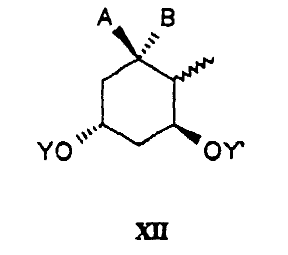 dans laquelle Y et Y' signifient chacun un atome d'hydrogène, un groupement alcanoyle ayant 1 à 9 atomes de carbone ou un groupement aroyle ou chacun un groupement alkyle ou aryle ou silyle substitué par un mélange aryle/alkyle ou un autre groupement protecteur courant,
et A et B signifient ensemble un groupement carbonyle libre ou protégé, les groupements protecteurs étant sélectionnés parmi dialkoxycétal, 1,3-dioxanne, 1,3-dioxolanne, dialkylhydrazone, tosylhydrazone, oxime, oximéther d'alkyle ou de benzyle ou A ou B peut être un atome d'hydrogène et l'autre groupement correspondant B ou A peut être un groupement hydroxyle, lequel peut être libre ou protégé, les groupements protecteurs étant sélectionnés parmi benzyléther, p-méthoxybenzyléther, o-, m-, p-nitrobenzyléther, éther-TBDMS, éther-TIPS, éther-TBDPS, éther-TES, éther-MOM, éther-MEM, éther-SEM, éther-EE, éther-THP et éther-THF.

12. Composé suivant la revendication 11, à savoir
(1*S*,3*S*)-4-méthyl-5-(phénylméthoxy)-1,3-cyclohexandiol;
(1*S*,5*R*)-1,5-bis[[diméthyl(1,1-diméthyléthyl)silyl]oxy]-2-méthyl-3-(phénylméthoxy)cyclohexane;
(3*S*,5*R*)-3,5-bis[[diméthyl(1,1-diméthyléthyl)silyl]oxy]-2-méthylcyclohexanol;
(3*S*,5*R*)-3,5-bis(acétyloxy)-2-méthylcyclohexanol;
(*E*)-(3*S*,5*R*)-3,5-dihydroxy-2-méthylcyclohexanonoxime;
(*Z*)-(3*S*,5*R*)-3,5-dihydroxy-2-méthylcyclohexanonoxime;
(*E*)-(3*S*,5*R*)-3,5-dihydroxy-2-méthylcyclohexanone-O-méthyloxime;
(*Z*)-(3*S*,5*R*)-3,5-dihydroxy-2-méthylcyclohexanone-O-méthyloxime;
(*E*)-(3*S*,5*R*)-3,5-bis[[diméthyl(1,1-diméthyléthyl)silyl]oxy]-2-méthylcyclohexanonoxime;
(*Z*)-(3*S*,5*R*)-3,5-bis[[diméthyl(1,1-diméthyléthyl)silyl]oxy]-2-méthylcyclohexanonoxime;
(*E*)-(3*S*,5*R*)-3,5-bis[[diméthyl(1,1-diméthyléthyl)silyl]oxy]-2-méthylcyclohexanone-O-méthyloxime;
(*Z*)-(3*S*,5*R*)-3,5-bis[[diméthyl(1,1-diméthyléthyl)silyl]oxy]-2-méthylcyclohexanone-O-méthyloxime;
(1*S*,5*S*)-1,5-bis(acétyloxy)-3-[[(1,1-diméthyléthyl)diphénylsilyl]oxy]-2-méthylcyclohexane;
(1*S*,3*S*)-5-[[(1,1-diméthyléthyl)diphénylsilyl]oxy]4-méthyl-1,3-cyclohexandiol;
(1*S*,5*S*)-1,5-bis(1-éthoxyéthoxy)-3-[[(1,1-diméthyléthyl)diphénylsilyl]oxy]-2-méthylcyclohexane;
(3*S*,5*R*)-3,5-bis(1-éthoxyéthoxy)-2-méthylcyclohexanol.
